# EUROPEAN PATENT SPECIFICATION

(11) **EP 4 379 350 B1**
(45) Date of publication and mention of the grant of the patent: **28.01.2026**
(21) Application number: 23210251.7
(22) Date of filing: 16.11.2023
(51) Int. Cl.: G01N 15/14, G01N 33/493, G01N 15/1429, G01N 15/01, G01N 15/10, G01N 15/1433

(54) **PARTICLE DETECTION METHOD, PARTICLE DETECTION APPARATUS, AND PROGRAM**
PARTIKELDETEKTIONSVERFAHREN, PARTIKELDETEKTIONSVORRICHTUNG UND PROGRAMM
PROCÉDÉ DE DÉTECTION DE PARTICULES, APPAREIL DE DÉTECTION DE PARTICULES ET PROGRAMME

(30) Priority: 22.11.2022 JP 2022186333
(43) Date of publication of application: 05.06.2024
(73) Proprietor: SYSMEX CORPORATION, Kobe-shi Hyogo 651-0073 (JP)
(72) Inventor: MASAGO, Akinori, Kobe-shi, Hyogo, 651-0073 (JP); TSUBOI, Kazuya, Nagoya-shi, Aichi, 453-0801 (JP)
(74) Representative: Becker Kurig & Partner Patentanwälte mbB

(56) References cited:
- US-A1- 2019 049 379
- US-B2- 11 060 134
- YOKOYAMA TAKASHI: "A standardized protocol for the mulberry cells and mulberry bodies in the urinary sediment of Fabry disease", TOIN UNIVERSITY OF YOKOHAMA RESEARCH BULLETIN, vol. 43, 31 December 2020 (2020-12-31), pages 83 - 90, XP093155204, Retrieved from the Internet <URL:https://toin.repo.nii.ac.jp/records/410>

## Description

### CROSS REFERENCE TO RELATED APPLICATIONS

This application claims priority from prior Japanese Patent Application No. 2022-186333, filed on November 22, 2022, entitled "PARTICLE DETECTION METHOD, PARTICLE DETECTION APPARATUS, AND PROGRAM".

### FIELD OF THE INVENTION

The present invention relates to a particle detection method, a particle detection apparatus, and a program.

### BACKGROUND OF THE INVENTION

Fabry disease is one type of genetic disease and is developed through accumulation of a lipid called globotriaosylceramide (Gb3) in lysosomes which are places for digestion within cells. When Fabry disease is developed, the lysosomes malfunction so that various organs experience disorders. Although the causal relationship between development of Fabry disease and urinary mulberry bodies has been conventionally known, since the amount of urinary mulberry bodies contained in a urinary sediment is very small, it is difficult to detect the mulberry bodies through a urinary sediment test.

Further, although urinary mulberry bodies have been visually checked by using a microscope, since the forms of urinary mulberry bodies are similar to the forms of red blood cells and fungi, it is highly difficult to distinguish these forms through this method. Therefore, examiners are required to have advanced skills to achieve this distinguishment.

Tomoko Namba, "Nyoukensa de Kisyoushikkan no Soukishindan ga Kanou ni" (provisional translation: Enabling Early Diagnosis of Rare Disease through Urine Test), [online], January 8, 2021, ResOU, [searched on October 14, 2022], the Internet <URL: https://resou.osaka-u.ac.jp/ja/research/2021/20210108_4> describes that staining of a urinary sediment has made it clear that urinary mulberry bodies are derived from lysosomes which are present in the cytoplasm of a podocyte and in which Gb3 has been accumulated.

In addition, Japanese Laid-Open Patent Publication No. 2022-59094 describes a method in which a urinary sediment image is applied to a neural network so that whether or not mulberry bodies are contained in a urine specimen of a subject is determined.

Gb3 is expressed with respect to not only mulberry bodies but also tubular cells. Therefore, although the above Tomoko Namba, "Nyoukensa de Kisyoushikkan no Soukishindan ga Kanou ni" (provisional translation: Enabling Early Diagnosis of Rare Disease through Urine Test), [online], January 8, 2021, ResOU, [searched on October 14, 2022], the Internet <URL: https://resou.osaka-u.ac.jp/ja/research/2021/20210108_4> has clarified that urinary mulberry bodies are derived from lysosomes in which Gb3 has been accumulated, it is difficult to accurately distinguish mulberry bodies and tubular cells from each other by utilizing Gb3.

In addition, the number of patients who suffer from Fabry disease is very small, and furthermore, the number of mulberry bodies contained in urine specimens of these patients is also very small. Therefore, with the method described in the above Japanese Laid-Open Patent Publication No. 2022-59094, a sufficient number of pieces of training data for training the neural network cannot be used, and it is difficult to put this method into practical use.
Document Yokoyama Takashi : "A standardization protocol for the mulberry cells and mulberry bodies in the urinary sediment of Fabry desease", Toin University of Yokohama research Bulleting, vol. 43, pages 83-90, relates to a non-invasive standardization protocol to identify and quantitatively evaluate the presence of urinary mulberry cells or mulberry bodies of Fabry disease.
Document US 11 060 134 B2 relates to a cell selection method including a sample preparation step of preparing a sample by performing staining of nucleic acid in each of cells by a first fluorescent dye; and hybridization with respect to an evaluation target region in DNA in each cell by an evaluation probe including a second fluorescent dye; a light receiving step of applying light to the sample and receiving fluorescence from the first fluorescent dye and fluorescence from the second fluorescent dye; and a selection step of selecting an analysis target cell on the basis of intensity of the fluorescence from the first fluorescent dye and intensity of the fluorescence from the second fluorescent dye, wherein the first fluorescent dye is a dye that emits fluorescence having a first wavelength, and the second fluorescent dye is a dye that emits fluorescence having a second wavelength different from the first wavelength.
Document US 2019/049379 A1 relates to a sample analyzer that includes: a measurement unit configured to apply light to a measurement specimen containing a plurality of kinds of target substances each labeled with fluorescence, and detect a plurality of kinds of fluorescences having different wavelengths; and a processing unit configured to analyze the plurality of kinds of target substances based on a detection result from the measurement unit, and information, on a color of fluorescence, which is set to be variable so as to correspond to the plurality of kinds of target substances.

In view of these problems, an object of the present invention is to provide a particle detection method, a particle detection apparatus, and a program that enable mulberry bodies to be more accurately extracted.

### SUMMARY OF THE INVENTION

A particle detection method of the present invention includes: preparing a sample containing a urine specimen collected from a subject, a first fluorescent dye that stains a nucleic acid, an antibody that specifically binds to a red blood cell, and a second fluorescent dye that binds to the antibody that specifically binds to the red blood cell (S1); imaging each of a plurality of particles in the sample, to obtain fluorescence images (S3); and extracting a particle regarding which information obtained from the corresponding fluorescence image is included in a range (R2) corresponding to mulberry bodies (S5).

With the particle detection method of the present invention, the particle regarding which the information obtained from the corresponding fluorescence image is included in the range corresponding to mulberry bodies is extracted, whereby mulberry bodies can be more accurately extracted.

A particle detection apparatus (1) of the present invention includes: a sample preparation unit (18) configured to prepare a sample containing a urine specimen collected from a subject, a first fluorescent dye that stains a nucleic acid, an antibody that specifically binds to a red blood cell, and a second fluorescent dye that binds to the antibody that specifically binds to the red blood cell; an imaging unit (19) configured to image each of a plurality of particles in the sample, to obtain fluorescence images; and a processing unit (11) configured to extract a particle regarding which information obtained from the corresponding fluorescence image is included in a range (R2) corresponding to mulberry bodies.

With the particle detection apparatus of the present invention, the particle regarding which the information obtained from the corresponding fluorescence image is included in the range corresponding to mulberry bodies is extracted, whereby mulberry bodies can be more accurately extracted.

A program (12a) of the present invention is a program configured to cause a computer to execute a process of detecting a particle in a urine specimen collected from a subject, the process including extracting a particle regarding which information obtained from a corresponding fluorescence image is included in a range (R2) corresponding to mulberry bodies, the corresponding fluorescence image being obtained by imaging each of a plurality of particles in a sample containing the urine specimen, a first fluorescent dye that stains a nucleic acid, an antibody that specifically binds to a red blood cell, and a second fluorescent dye that binds to the antibody that specifically binds to the red blood cell.

With the program of the present invention, the particle regarding which the information obtained from the corresponding fluorescence image is included in the range corresponding to mulberry bodies is extracted, whereby mulberry bodies can be more accurately extracted.

According to the present invention, mulberry bodies can be more accurately extracted.

### BRIEF DESCRIPTION OF THE DRAWINGS

FIG. 1 is a block diagram showing functions of a particle detection apparatus, according to an embodiment;
FIG. 2 schematically shows a configuration of an imaging unit, according to the embodiment;
FIG. 3 is a flowchart showing a process regarding a particle detection method to be performed by a processing unit, according to the embodiment;
FIG. 4 shows a scattergram generated in a first extraction step, according to the embodiment;
FIG. 5 shows exemplary bright field images of particles plotted in a range other than a range for extraction on the scattergram in the first extraction step, according to the embodiment;
FIG. 6 shows a scattergram generated in a second extraction step, according to the embodiment;
FIG. 7 shows exemplary bright field images, first fluorescence images, and second fluorescence images of red blood cells, urothelial cells, and tubular epithelial cells plotted in ranges other than a range for extraction on the scattergram in the second extraction step, according to the embodiment;
FIG. 8 shows exemplary bright field images, first fluorescence images, and second fluorescence images of white blood cells and fungi plotted at and near positions other than positions in the range for extraction on the scattergram in the second extraction step, according to the embodiment;
FIG. 9 shows a scattergram generated in a third extraction step, according to the embodiment;
FIG. 10 shows exemplary bright field images of particles plotted at and near positions other than positions in a range for extraction on the scattergram in the third extraction step, according to the embodiment;
FIG. 11 shows exemplary bright field images, first fluorescence images, second fluorescence images, and third fluorescence images of particles plotted in the range for extraction on the scattergram in the third extraction step, according to the embodiment;
FIG. 12 shows a table indicating the number of particles included in each of the ranges for the respective first to third extraction steps, according to the embodiment;
FIG. 13 shows scattergrams obtained in the first to third extraction steps with respect to urine specimens shown in FIG. 12, according to the embodiment;
FIG. 14 shows scattergrams obtained in the first to third extraction steps with respect to urine specimens shown in FIG. 12, according to the embodiment;
FIG. 15 shows scattergrams obtained in the first to third extraction steps with respect to urine specimens shown in FIG. 12, according to the embodiment;
FIG. 16 shows scattergrams obtained in the first to third extraction steps with respect to urine specimens shown in FIG. 12, according to the embodiment;
FIG. 17 shows scattergrams obtained in the first to third extraction steps with respect to urine specimens shown in FIG. 12, according to the embodiment;
FIG. 18 shows scattergrams obtained in the first to third extraction steps with respect to urine specimens shown in FIG. 12, according to the embodiment;
FIG. 19 schematically shows a configuration of a screen in which images of particles are displayed, according to the embodiment;
FIG. 20 schematically shows a configuration of the screen in which the images of the particles are displayed, according to the embodiment;
FIG. 21 schematically shows a configuration of the screen in which an enlarged image region is displayed, according to the embodiment;
FIG. 22 shows exemplary bright field images of particles determined to be mulberry bodies by an operator, according to the embodiment;
FIG. 23 schematically shows a configuration of an enlarged image region according to a modification of the embodiment;
FIG. 24 schematically shows a configuration of a screen in which histories of and change in counting results of mulberry bodies regarding a same subject are displayed, according to the embodiment;
FIG. 25 schematically shows a configuration of a screen in which images of particles are displayed, according to a modification of the embodiment;
FIG. 26 is a flowchart showing a process regarding a particle detection method to be performed by a processing unit in a case of performing only the second extraction step as an extraction step, according to a first modification of a particle detection apparatus, method, and program of the embodiment;
FIG. 27 shows an example in which the first extraction step is performed by using only values related to sizes of particles based on bright field images, according to a second modification of the particle detection apparatus, method, and program of the embodiment;
FIG. 28 shows an example in which the first extraction step is performed by using only values related to focus states of the particles based on the bright field images, according to the second modification of the particle detection apparatus, method, and program of the embodiment;
FIG. 29 shows an example in which the third extraction step is performed by using only values related to contrasts of particles based on bright field images, according to a third modification of the particle detection apparatus, method, and program of the embodiment; and
FIG. 30 shows an example in which the third extraction step is performed by using only values related to variations in luminances of the particles, according to the third modification of the particle detection apparatus, method, and program of the embodiment.

### DETAILED DESCRIPTION

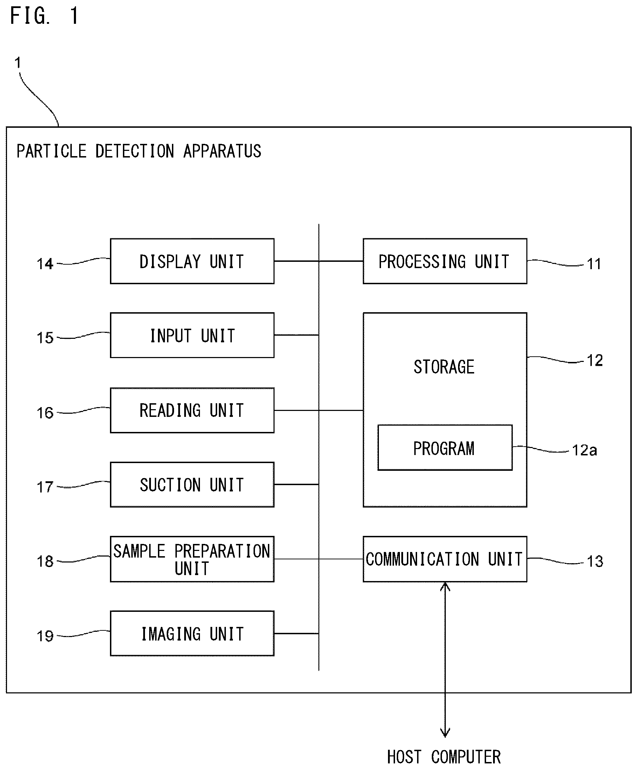

FIG. 1 is a block diagram showing functions of a particle detection apparatus 1.

The particle detection apparatus 1 is an apparatus for detecting particles contained in a urine specimen collected from a subject. The particle detection apparatus 1 includes a processing unit 11, a storage 12, a communication unit 13, a display unit 14, an input unit 15, a reading unit 16, a suction unit 17, a sample preparation unit 18, and an imaging unit 19.

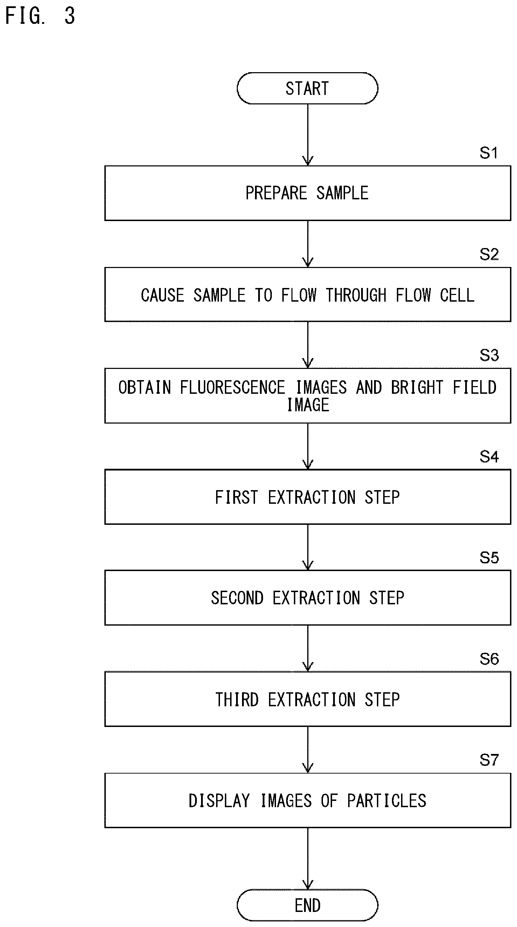

The processing unit 11 is implemented by, for example, an FPGA or a CPU. The processing unit 11 receives a signal outputted from each unit of the particle detection apparatus 1 and controls each unit of the particle detection apparatus 1. The storage 12 is implemented by, for example, an HDD or an SSD. The storage 12 stores therein a program 12a which causes the processing unit 11 to execute a process described later with reference to FIG. 3. The processing unit 11 reads the program 12a stored in the storage 12 and executes the process shown in FIG. 3 according to the program 12a. The program 12a may be stored in the storage 12 via a storing medium or may be stored in the storage 12 by being received from another computer via the communication unit 13.

The communication unit 13 is, for example, a communication interface based on the Ethernet standard. Via the communication unit 13, the processing unit 11 receives subject information corresponding to a specimen ID or the like from a host computer and transmits a counting result of particles or the like to the host computer.

The display unit 14 is implemented by, for example, a liquid crystal display. The input unit 15 is implemented by, for example, a keyboard, a pointing device including a mouse or a touch panel, etc. An operator operates the input unit 15 to operate operation sections such as a button in a screen displayed on the display unit 14. The display unit 14 and the input unit 15 may be integrated with each other. For example, the particle detection apparatus 1 may include a touch-panel type display as the display unit 14 and the input unit 15.

The reading unit 16 is implemented by, for example, a barcode reader. The reading unit 16 obtains a specimen ID by reading a barcode from a barcode label provided on a container containing a urine specimen. The container from which the specimen ID has been read is set in the particle detection apparatus 1.

The suction unit 17 suctions the urine specimen through a nozzle from the container having been set in the particle detection apparatus 1. The sample preparation unit 18 prepares a sample containing the urine specimen, a first fluorescent dye that stains a nucleic acid, an antibody that specifically binds to a red blood cell, a second fluorescent dye that binds to the antibody that specifically binds to the red blood cell, and a globotriaosylceramide (Gb3) antibody labeled with a third fluorescent dye. Consequently, the nucleic acid of each of particles in the urine specimen is stained with the first fluorescent dye, each of red blood cells in the urine specimen is stained with the second fluorescent dye via the antibody, and Gb3 in the urine specimen is stained with the third fluorescent dye.

The first fluorescent dye is composed so as to, when being irradiated with a light having a wavelength λ11, generate a fluorescence having a wavelength λ21. The second fluorescent dye is composed so as to, when being irradiated with a light having a wavelength λ12, generate a fluorescence having a wavelength λ22. The third fluorescent dye is composed so as to, when being irradiated with a light having a wavelength λ13, generate a fluorescence having a wavelength λ23. The wavelengths λ11, λ12, and λ13 are in wavelength bands different from one another in the present embodiment, but may be in wavelength bands identical to one another.

Here, the sample preparation unit 18 performs sample preparation such that the sample contains the Gb3 antibody labeled with the third fluorescent dye. However, since the fluorescence generated by the third fluorescent dye is not particularly necessary in an extraction step for mulberry bodies described later, the Gb3 antibody labeled with the third fluorescent dye does not have to be mixed in sample preparation.

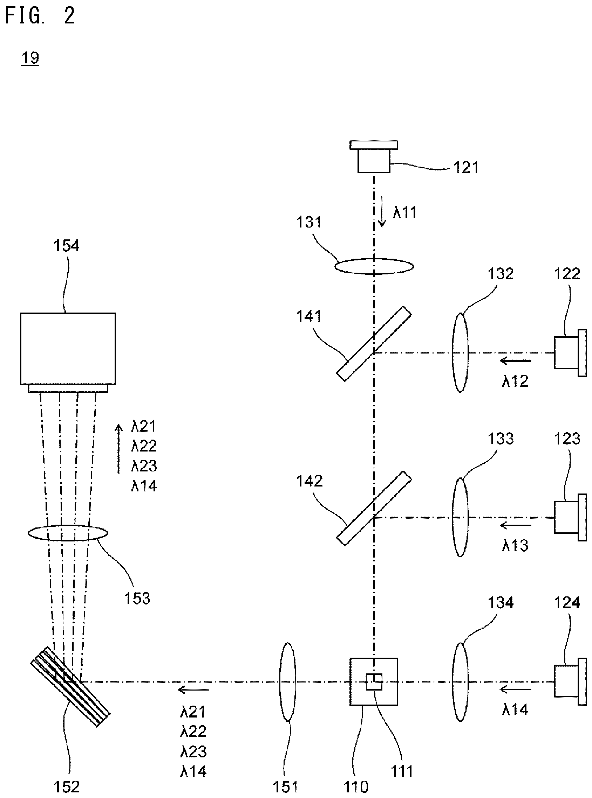

FIG. 2 schematically shows a configuration of the imaging unit 19.

The imaging unit 19 includes a flow cell 110, light sources 121 to 124, condenser lenses 131 to 134, dichroic mirrors 141 and 142, a condenser lens 151, an optical unit 152, a condenser lens 153, and a camera 154. The sample prepared by the sample preparation unit 18 is caused to flow through a flow path 111 of the flow cell 110.

The light sources 121 to 124 emit lights to the sample flowing through the flow cell 110. The light sources 121 to 124 are semiconductor laser light sources. The lights emitted from the light sources 121 to 124 are laser lights having the wavelengths λ11 to λ14, respectively. The condenser lenses 131 to 134 condense the lights emitted from the light sources 121 to 124, respectively. The dichroic mirror 141 allows the light having the wavelength λ11 to be transmitted therethrough and reflects the light having the wavelength λ12. The dichroic mirror 142 allows the lights having the wavelengths λ11 and λ12 to be transmitted therethrough and reflects the light having the wavelength λ13. Consequently, the sample flowing through the flow path 111 of the flow cell 110 is irradiated with the lights having the wavelengths λ11 to λ14.

When the sample flowing through the flow cell 110 is irradiated with the lights having the wavelengths λ11 to λ13, fluorescences are generated from the fluorescent dyes that have stained the particles. Specifically, when the first fluorescent dye that has stained the nucleic acid is irradiated with the light having the wavelength λ11, the fluorescence having the wavelength λ21 is generated from the first fluorescent dye. In addition, when the second fluorescent dye that has bound to the red blood cell is irradiated with the light having the wavelength λ12, the fluorescence having the wavelength λ22 is generated from the second fluorescent dye. In addition, when the third fluorescent dye with which the Gb3 antibody has been labeled is irradiated with the light having the wavelength λ13, the fluorescence having the wavelength λ23 is generated from the third fluorescent dye. The wavelengths λ21, λ22, and λ23 are in, for example, wavelength bands of green, red, and yellow lights, respectively. When the sample flowing through the flow cell 110 is irradiated with the light having the wavelength λ14, this light is transmitted through the particles in the sample. The wavelength λ14 is in a wavelength band that is suitable for bright field images and that is wider than the wavelength bands for the wavelengths λ11 to λ13.

The condenser lens 151 condenses: the fluorescences having the wavelengths λ21 to λ23 and generated from the sample flowing through the flow path 111 of the flow cell 110; and the light having the wavelength λ14 and transmitted through the sample flowing through the flow path 111 of the flow cell 110. The optical unit 152 has a configuration in which four dichroic mirrors have been combined. The four dichroic mirrors of the optical unit 152 reflect the fluorescences having the wavelengths λ21 to λ23 and the light having the wavelength λ14 at angles slightly different from one another so that the fluorescences and the light are apart from one another on a light reception surface of the camera 154. The condenser lens 153 condenses the fluorescences having the wavelengths λ21 to λ23 and the light having the wavelength λ14.

The camera 154 is, for example, a time delay integration (TDI) camera. The camera 154 images the fluorescences having the wavelengths λ21 to λ23 and the light having the wavelength λ14 and outputs, as imaging signals, a first fluorescence image, a second fluorescence image, and a third fluorescence image respectively corresponding to the fluorescences having the wavelengths λ21 to λ23 and a bright field image corresponding to the light having the wavelength λ14. The processing unit 11 in FIG. 1 extracts mulberry bodies contained in the urine specimen, by using the first fluorescence images, the second fluorescence images, and the bright field images taken by the camera 154.

Meanwhile, the third fluorescence image is an index for ascertaining the extent to which the particle contains Gb3, as described later. In the present embodiment, the third fluorescence image is not used for extracting mulberry bodies. Therefore, the imaging unit 19 in FIG. 2 does not have to be provided with any of the light source 123, the condenser lens 133, and the dichroic mirror 142.

FIG. 3 is a flowchart showing a process regarding a particle detection method to be performed by the processing unit 11.

The processing unit 11 executes the program 12a stored in the storage 12, to perform the process shown in FIG. 3.

In step S1, the processing unit 11 controls the sample preparation unit 18 to prepare a sample containing a urine specimen, a first fluorescent dye that stains a nucleic acid, an antibody that specifically binds to a red blood cell, a second fluorescent dye that binds to the antibody, and a Gb3 antibody labeled with a third fluorescent dye.

In step S2, the processing unit 11 controls the sample preparation unit 18 to cause the sample prepared in step S1 to flow through the flow path 111 of the flow cell 110. In step S3, the processing unit 11 controls the light sources 121 to 124 to emit the lights to the sample flowing through the flow path 111 and controls the camera 154 to image each of a plurality of particles in the sample, i.e., to image the fluorescences having the wavelengths λ21 to λ23 and generated from the sample and the light having the wavelength λ14 and transmitted through the sample. Consequently, the processing unit 11 obtains, for each of the particles in the sample, first to third fluorescence images and a bright field image. The first fluorescence image is a color image taken with exclusion of wavelength bands other than the band for the green color, the second fluorescence image is a color image taken with exclusion of wavelength bands other than the band for the red color, the third fluorescence image is a color image taken with exclusion of wavelength bands other than the band for the yellow color, and the bright field image is a gray scale image.

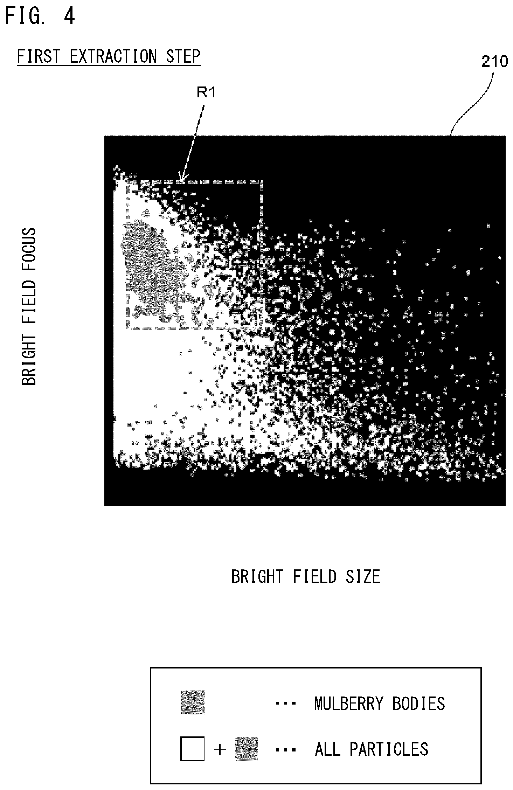

In step S4, the processing unit 11 performs a first extraction step regarding mulberry bodies on the basis of the bright field images of all the particles obtained in step S3. In step S5, the processing unit 11 performs a second extraction step regarding mulberry bodies on the basis of the first fluorescence images and the second fluorescence images of particles extracted through the first extraction step in step S4. In step S6, the processing unit 11 performs a third extraction step regarding mulberry bodies on the basis of the bright field images of particles extracted through the second extraction step in step S5. The first to third extraction steps will be described later in detail with reference to FIG. 4 to FIG. 11.

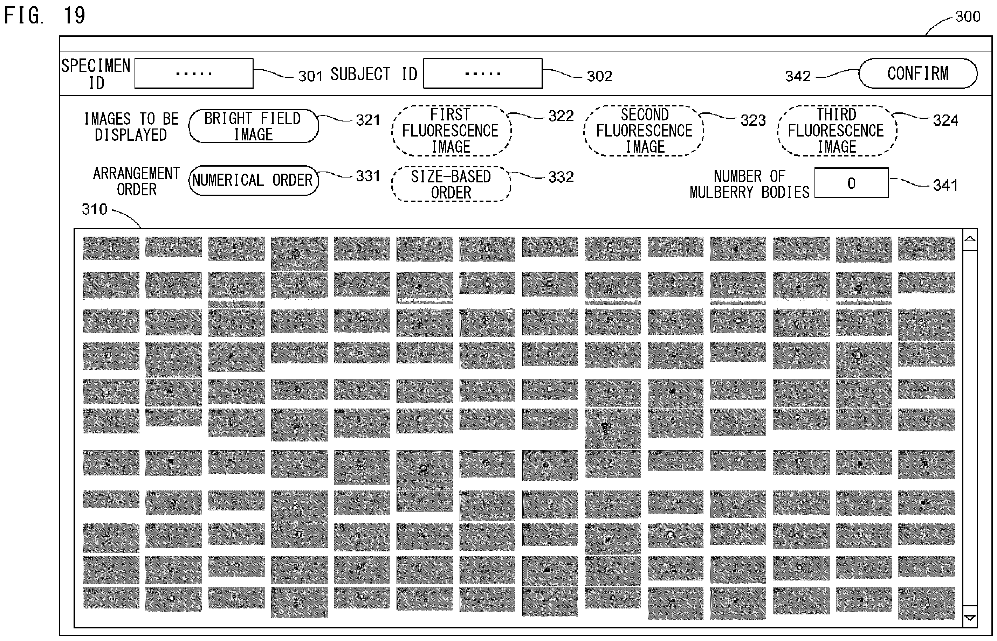

In step S7, the processing unit 11 causes the display unit 14 to display images of the particles extracted through the third extraction step in step S6. Displaying of the images of the particles will be described later with reference to FIG. 19 to FIG. 25. The sample prepared in step S1 does not have to contain the Gb3 antibody labeled with the third fluorescent dye. In step S3, the third fluorescence images do not have to be obtained. The processing in step S6 may be omitted.

Next, the first to third extraction steps regarding mulberry bodies will be described with reference to FIG. 4 to FIG. 11.

Although the extraction steps are described below as steps of generating scattergrams 210, 220, and 230 by the processing unit 11 at the time of extraction as shown in FIGS. 4, 6, and 9, scattergrams do not necessarily have to be generated. That is, the processing unit 11 may perform, through data processing based on a similar procedure, the process of generating scattergrams and extracting the particles in predetermined ranges.

FIG. 4 shows the scattergram 210 generated in the first extraction step.

Regarding the bright field images of all the particles obtained by the camera 154, the processing unit 11 obtains a bright field size and a bright field focus from each of these bright field images.

The bright field size is an example of a value related to a size of the corresponding particle based on the bright field image and is morphological information about the particle. The bright field size is, for example, the number of pixels identified as constituting the region of the particle in the bright field image. The bright field focus is an example of a value related to a focus state of the particle based on the bright field image. The bright field focus is, for example, a value (average gradient) obtained by: normalizing the values of the pixels identified as constituting the region of the particle in the bright field image; and dividing the total value of gradients of the values of the pixels between adjacent ones of the pixels by the number of the pixels. A bright field focus having a larger value leads to a state where the particle is better focused in the bright field image.

The processing unit 11 plots all the particles onto the scattergram 210 by using the bright field sizes and the bright field foci obtained from the bright field images. The scattergram 210 has a horizontal axis indicating the bright field size and a vertical axis indicating the bright field focus.

The processing unit 11 extracts, from the scattergram 210, particles included in a range R1 corresponding to mulberry bodies. The range R1 is a fixed range that is prestored in the storage 12. The range in the horizontal axis direction of the range R1 is set to a range, of bright field sizes, in which mulberry bodies have a high probability of being contained and in which a single bacterium, urothelial cells having large sizes, squamous epithelial cells, and the like can be excluded. The range in the vertical axis direction of the range R1 is set to a range, of bright field foci, in which particles that are not focused in the bright field images can be excluded. The upper limit in the vertical axis direction of the range R1 does not have to be set. Upon extraction of the particles in the range R1, the first extraction step is ended.

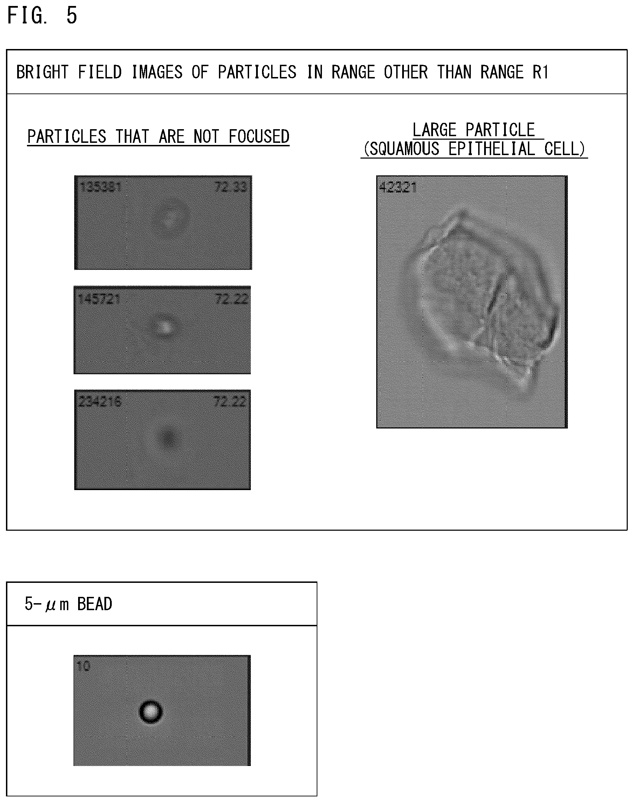

On the upper stage of FIG. 5, exemplary bright field images of particles plotted in a range other than the range R1 on the scattergram 210 in FIG. 4 are shown.

As shown in the three bright field images shown on the left side of the upper stage of FIG. 5, when particles are not focused, the particles shown in the corresponding bright field images are blurred. Therefore, these bright field images cannot be used for the subsequent extraction. The particles that are not focused as described above are distributed outside the range R1, and thus, are excluded through the first extraction step. In addition, a squamous epithelial cell having a size that is much larger than the size of a mulberry body, is shown in the bright field image shown on the right side of the upper stage of FIG. 5. When the size of a particle is much larger than the size of an ordinary mulberry body as shown in the bright field image shown on the right side of the upper stage of FIG. 5, the particle has a low probability of being a mulberry body. Particles having sizes that are much larger than the size of an ordinary mulberry body as described above are distributed outside the range R1, and thus, are excluded through the first extraction step.

In addition, a bright field image obtained by imaging a bead having a size of 5 µm that is approximately equal to the size of a mulberry body by the particle detection apparatus 1 is shown on the lower stage of FIG. 5. When the range R1 is preset, the ranges of bright field foci and bright field sizes of the range R1 can be set by using, for example, bright field images obtained by imaging beads having predetermined sizes in this manner.

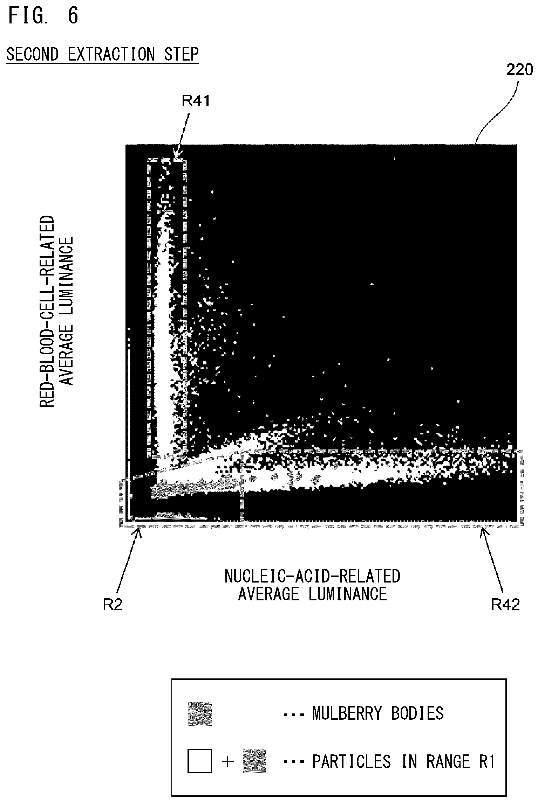

FIG. 6 shows the scattergram 220 generated in the second extraction step.

The processing unit 11 obtains a nucleic-acid-related average luminance and a red-blood-cell-related average luminance respectively from the first fluorescence image and the second fluorescence image of each of the particles in the range R1 extracted through the first extraction step.

The nucleic-acid-related average luminance is an example of a value related to the amount of the first fluorescence generated from the particle by the first fluorescent dye. The nucleic-acid-related average luminance is, for example, the average of the luminance values of green components out of pixels identified as constituting the region of the particle in the first fluorescence image. The red-blood-cell-related average luminance is an example of a value related to the amount of the second fluorescence generated from the particle by second fluorescent dye. The red-blood-cell-related average luminance is, for example, the average of the luminance values of red components out of pixels identified as constituting the region of the particle in the second fluorescence image.

Although the value related to the amount of the first fluorescence generated from the particle by the first fluorescent dye is the nucleic-acid-related average luminance in the present embodiment, the value is not limited to the nucleic-acid-related average luminance. For example, the value may be the total value, the maximum value, the intermediate value, or the like of the luminance values of the green components out of the pixels identified as constituting the region of the particle in the first fluorescence image. Likewise, although the value related to the amount of the second fluorescence generated from the particle by the second fluorescent dye is the red-blood-cell-related average luminance in the present embodiment, the value is not limited to the red-blood-cell-related average luminance. For example, the value may be the total value, the maximum value, the intermediate value, or the like of the luminance values of the red components out of the pixels identified as constituting the region of the particle in the second fluorescence image.

The processing unit 11 plots all the particles in the range R1 extracted through the first extraction step onto the scattergram 220 by using the nucleic-acid-related average luminances obtained from the first fluorescence images and the red-blood-cell-related average luminances obtained from the second fluorescence images. The scattergram 220 has a horizontal axis indicating the nucleic-acid-related average luminance and a vertical axis indicating the red-blood-cell-related average luminance.

The processing unit 11 extracts, from the scattergram 220, particles included in a range R2 corresponding to mulberry bodies. The range R2 is a fixed range that is prestored in the storage 12. In FIG. 6, a range R41 is a range corresponding to red blood cells. A range R42 is a range corresponding to fungi. The range R42 also includes, in addition to fungi, urothelial cells, tubular epithelial cells, and white blood cells. The range R2 is a range overlapping with neither the range R41 as the range corresponding to red blood cells nor the range R42 as the range corresponding to fungi. That is, the range R2 is set to a range in which the red blood cells distributed in the range R41 and the urothelial cells, the tubular epithelial cells, the white blood cells, and the fungi distributed in the range R42 can be excluded. Upon extraction of the particles in the range R2, the second extraction step is ended.

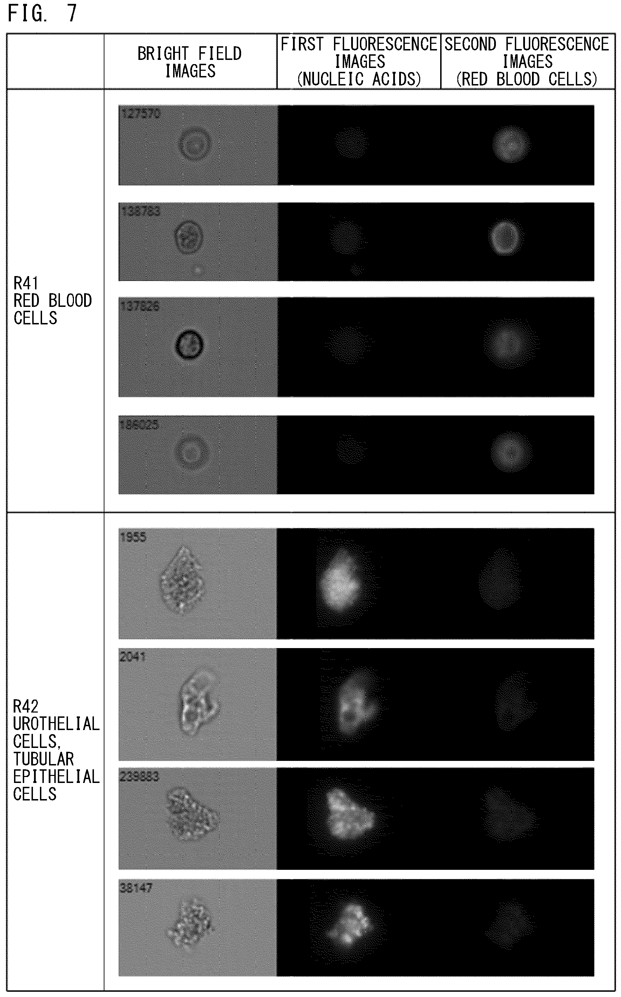

On the upper stage of FIG. 7, exemplary bright field images, first fluorescence images, and second fluorescence images of red blood cells plotted in the range R41 on the scattergram 220 in FIG. 6 are shown.

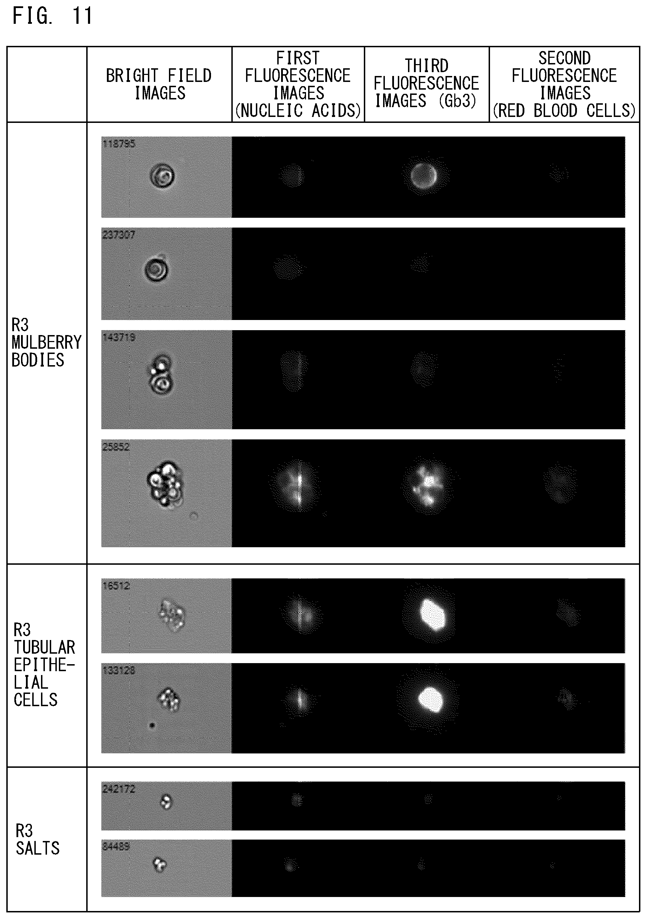

The red blood cells in the bright field images are shown as ones having circular shapes and are morphologically similar to mulberry bodies. Therefore, it is difficult to distinguish mulberry bodies (see FIG. 11) and the red blood cells from each other by visually checking the bright field images. However, since red blood cells have no nuclei, the first fluorescence images of the red blood cells hardly show the fluorescence from the first fluorescent dye. Meanwhile, since the red blood cells are labeled with the second fluorescent dye, the second fluorescence images of the red blood cells clearly show the fluorescence from the second fluorescent dye. Therefore, the red blood cells are distributed in the range R41 which does not overlap with the range R2 in FIG. 6, whereby the red blood cells are excluded through the second extraction step.

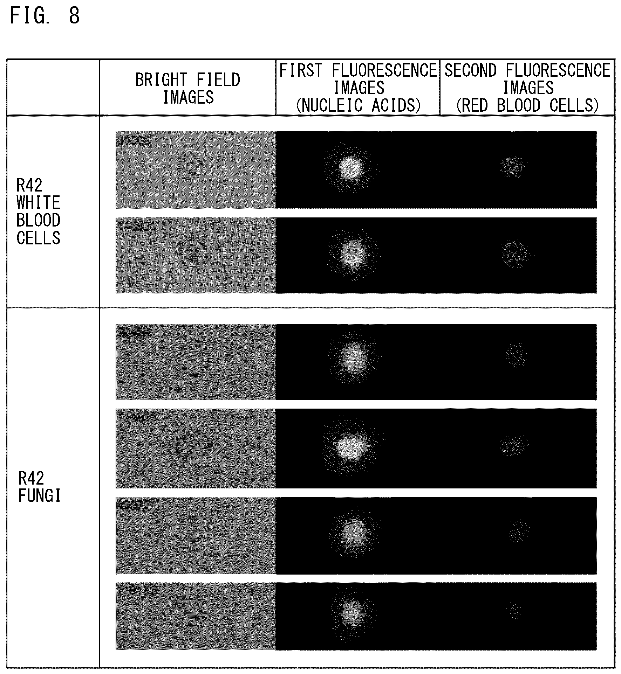

On the lower stage of FIG. 7 and in FIG. 8, exemplary bright field images, first fluorescence images, and second fluorescence images of urothelial cells, tubular epithelial cells, white blood cells, and fungi plotted in the range R42 on the scattergram 220 in FIG. 6 are shown.

Many of these cells in the bright field images are shown as ones having circular shapes, and thus it is difficult to distinguish mulberry bodies (see FIG. 11) and these cells from each other by visually checking the bright field images. In particular, fungi are morphologically similar to mulberry bodies, and thus it is difficult to distinguish mulberry bodies and fungi from each other through visual checking. However, since these cells have nuclei, the first fluorescence images of these cells clearly show the fluorescence from the first fluorescent dye. Meanwhile, since these cells are hardly labeled with the second fluorescent dye, the second fluorescence images of these cells hardly show the fluorescence from the second fluorescent dye. Therefore, these cells are distributed in and near the range R42 which does not overlap with the range R2 in FIG. 6, whereby these cells are excluded through the second extraction step.

Since mulberry bodies have no nuclei, the first fluorescence images of the mulberry bodies hardly show the fluorescence from the first fluorescent dye. In addition, since the mulberry bodies are hardly labeled with the second fluorescent dye, the second fluorescence images of the mulberry bodies hardly show the fluorescence from the second fluorescent dye. Therefore, the range R2 is set to a region in which: the value related to the amount of the second fluorescence is smaller than that in the range R41; and the value related to the amount of the first fluorescence is smaller than that in the range R42.

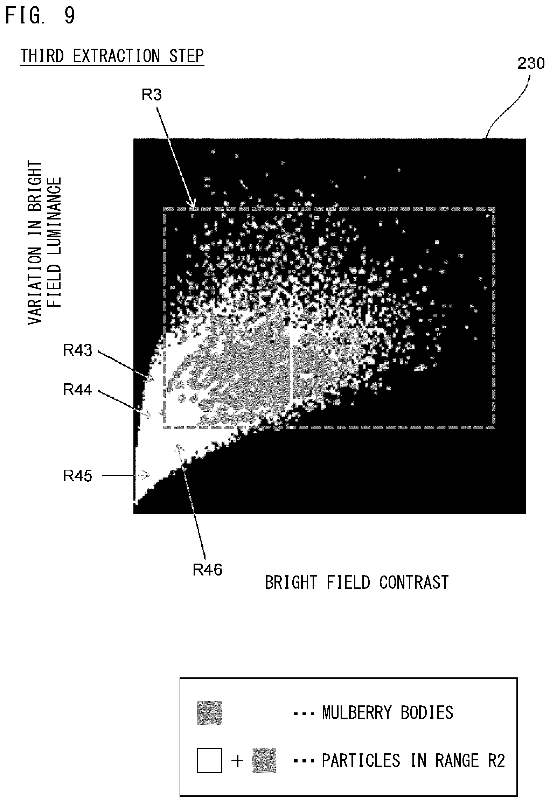

FIG. 9 shows the scattergram 230 generated in the third extraction step.

The processing unit 11 obtains a bright field contrast and a variation in bright field luminance from the bright field image of each of the particles in the range R2 extracted through the second extraction step.

The bright field contrast is an example of a value related to a contrast of the particle based on the bright field image and is morphological information about the particle. The bright field contrast is, for example, a value obtained by: within pixels identified as constituting the region of the particle in the bright field image, adding up gradients of the values of pixels in a pixel range composed of 3 by 3 pixels and performing shifts to different pixel ranges; calculating gradients of the values of pixels in the respective pixel ranges; and dividing the total gradient of the values of the pixels in all the pixel ranges by the number of the pixels constituting the region of the particle. The variation in bright field luminance is an example of a value related to a variation in a luminance of the particle based on the bright field image and is morphological information about the particle. The variation in bright field luminance is, for example, a standard deviation of the values of the pixels identified as constituting the region of the particle in the bright field image.

The bright field contrast indicates the amount of change in the values of the pixels in the bright field image. For example, in the bright field image, a larger difference in darkness/paleness between the edge and the inside of the particle leads to a larger bright field contrast. The bright field contrast varies according to the difference in darkness/paleness between the aggregate surface and the inside, the difference in darkness/paleness due to the shapes of spirals of mulberry bodies, or the like. The variation in bright field luminance indicates a state of distribution of the values of the pixels in the bright field image. For example, in the bright field image, a larger variation in the values of the pixels of the particle and a larger extent of non-uniformity among the values of the pixels lead to a larger variation in bright field luminance. Since many of mulberry bodies have the shapes of spirals, the bright field contrasts and the variations in bright field luminances of particles corresponding to mulberry bodies are comparatively large.

The processing unit 11 plots all the particles in the range R2 extracted through the second extraction step onto the scattergram 230 by using the bright field contrasts and the variations in bright field luminances obtained from the bright field images. The scattergram 230 has a horizontal axis indicating the bright field contrast and a vertical axis indicating the variation in bright field luminance.

The processing unit 11 extracts, from the scattergram 230, particles included in a range R3 corresponding to mulberry bodies. The range R3 is a fixed range that is prestored in the storage 12. The range R3 is set to a range in which particles indicated with only brightness/darkness and distributed at and near a position R43, debris distributed at and near a position R44, particles that are not focused and that are distributed at and near a position R45, and aggregates of small particles distributed at and near a position R46 can be excluded. Specifically, the range R3 is set to a region in which: the value related to the contrast is larger than that at the position R44 where debris appears; and the value related to the variation in the luminance is larger than that at the position R46 where aggregates of small particles appear. Upon extraction of the particles in the range R3, the third extraction step is ended.

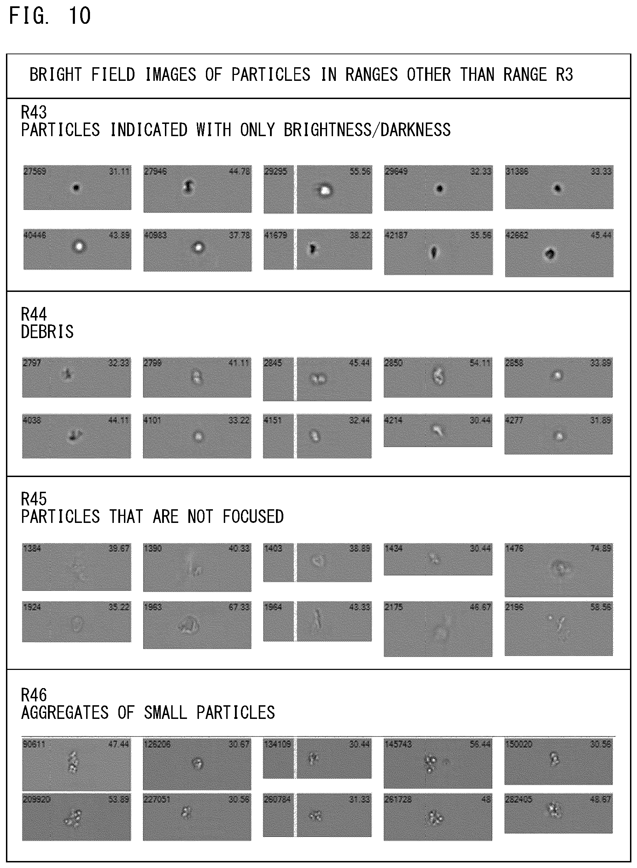

FIG. 10 shows exemplary bright field images of particles plotted at and near the positions R43, R44, R45, and R46 on the scattergram 230 in FIG. 9.

As shown on the first stage of FIG. 10, bright field images of particles at and near the position R43 show particles indicated with only brightness/darkness. The particles indicated with only brightness/darkness are distributed at and near the position R43 outside the range R3, and thus, are excluded through the third extraction step.

As shown on the second stage of FIG. 10, bright field images of particles at and near the position R44 show debris. The debris refers to, for example, pieces of necrosed tissues. The debris is distributed at and near the position R44 outside the range R3, and thus, is excluded through the third extraction step.

As shown on the third stage of FIG. 10, bright field images of particles at and near the position R45 show particles that are not focused. The particles that are not focused are distributed at and near the position R45 outside the range R3, and thus, are excluded through the third extraction step.

As shown on the fourth stage of FIG. 10, bright field images of particles at and near the position R46 show aggregates of small particles. The aggregates of small particles are distributed at and near the position R46 outside the range R3, and thus, are excluded through the third extraction step.

FIG. 11 shows exemplary bright field images, first fluorescence images, second fluorescence images, and third fluorescence images of particles plotted in the range R3 on the scattergram 230 in FIG. 9.

In the range R3, mulberry bodies, tubular epithelial cells, salts, and the like are distributed.

The bright field images for the range R3 include bright field images of mulberry bodies in which the shapes of spirals, double circles, white voids, and the like are formed. The mulberry bodies sometimes include one having the shape of an aggregate (mulberry) as shown in the bright field image, of a mulberry body, on the lowermost row. Since the mulberry bodies are hardly stained with the second fluorescent dye, the second fluorescence images of the mulberry bodies hardly show the fluorescence from the second fluorescent dye. In addition, since many of the mulberry bodies excluding mulberry bodies having the shapes of aggregates are hardly stained with the first fluorescent dye, the first fluorescence images of these mulberry bodies hardly show the fluorescence from the first fluorescent dye.

In the present embodiment, the third fluorescence images are not used for extracting mulberry bodies, but Gb3 in the urine specimen is labeled with the third fluorescent dye and the third fluorescence images are obtained, in order to check presence/absence of Gb3 in the particles.

With reference to the third fluorescence images of the mulberry bodies, Gb3 may be expressed with respect to the mulberry bodies, e.g., Gb3 may be expressed when the mulberry bodies have the shapes of aggregates. However, with reference to the third fluorescence images of the tubular epithelial cells, Gb3 may be expressed also with respect to the tubular epithelial cells. Therefore, it is found to be difficult to perform classification into mulberry bodies and tubular epithelial cells according to whether Gb3 has been expressed. Meanwhile, the extraction steps for mulberry bodies according to the embodiment are as follows. That is, although some of tubular epithelial cells remain in the range R3, many of the tubular epithelial cells are distributed in the range R42, and the range R42 does not overlap with the range R2 corresponding to mulberry bodies, on the scattergram 220 in FIG. 6. Consequently, many of the tubular epithelial cells can be excluded particularly through the second extraction step.

Among the mulberry bodies shown on the upper stage of FIG. 11, mulberry bodies in the second and third images from the uppermost image are hardly stained with the third fluorescent dye. Thus, sufficient visibility might not be attained in a conventional method for improving the visibility of mulberry bodies by using an antibody staining dye that specifically stains Gb3.

The tubular epithelial cells shown on the intermediate stage of FIG. 11, the salts shown on the lower stage of FIG. 11, and the like remain in the range R3, and the mulberry bodies included in the range R3 are identified by an operator through visual checking by using a screen 300 displayed on the display unit 14. The screen 300 will be described later with reference to FIG. 19 and the subsequent drawings. Through the first to third extraction steps, many particles other than mulberry bodies contained in the urine specimen are excluded, i.e., the particles included in the ranges R1, R2, and R3 corresponding to mulberry bodies are extracted. Consequently, the operator can efficiently identify mulberry bodies with reference to images displayed in the screen 300 described later.

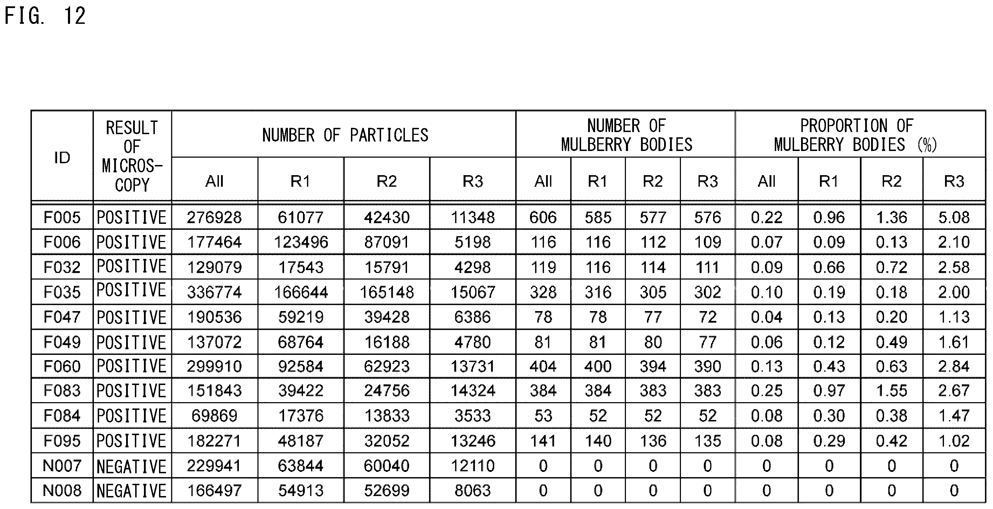

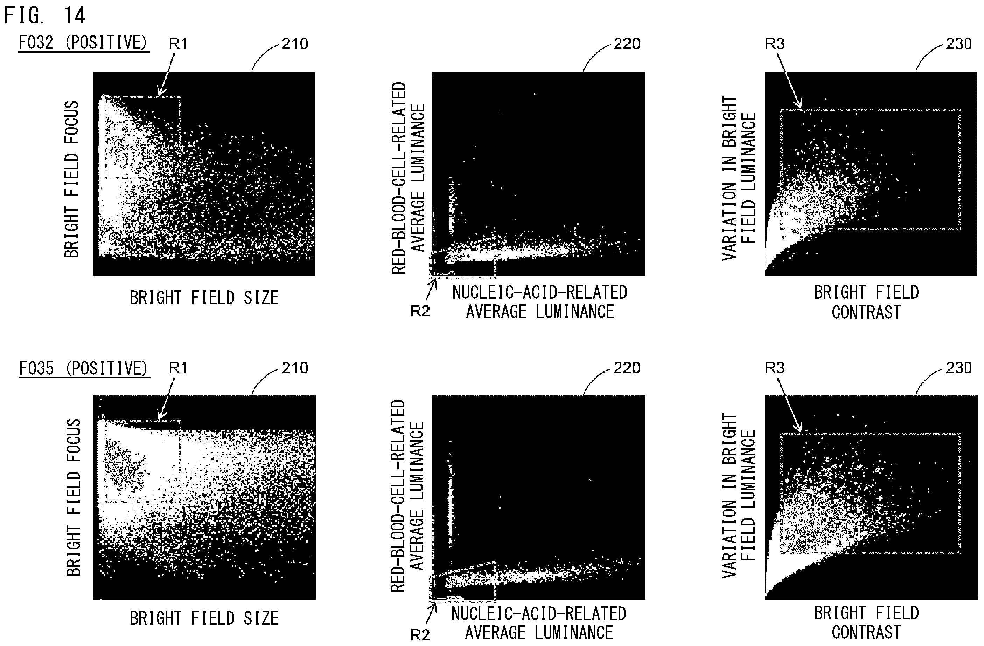

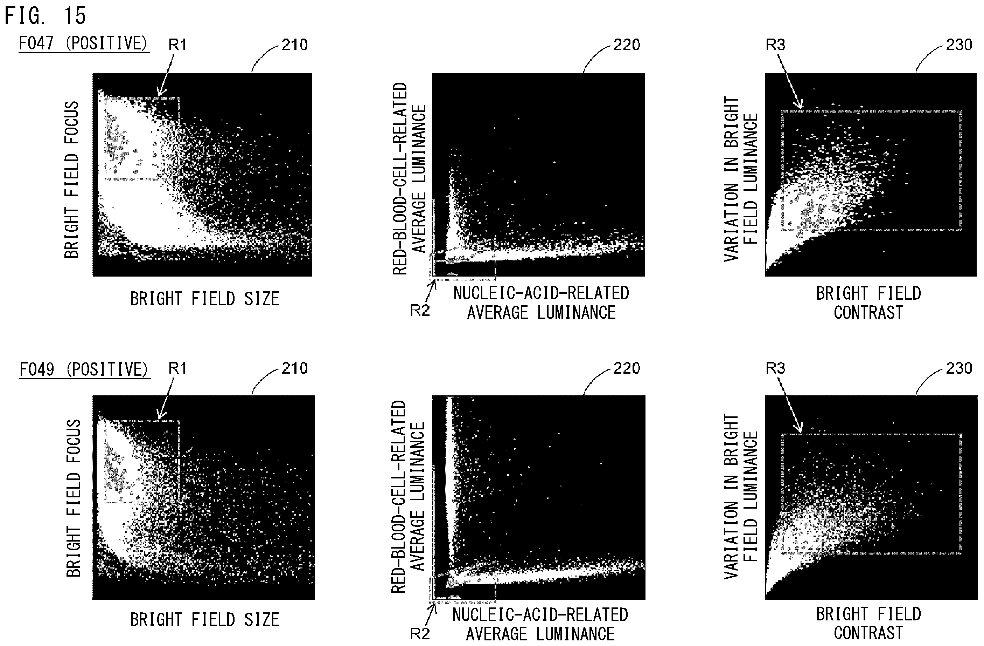

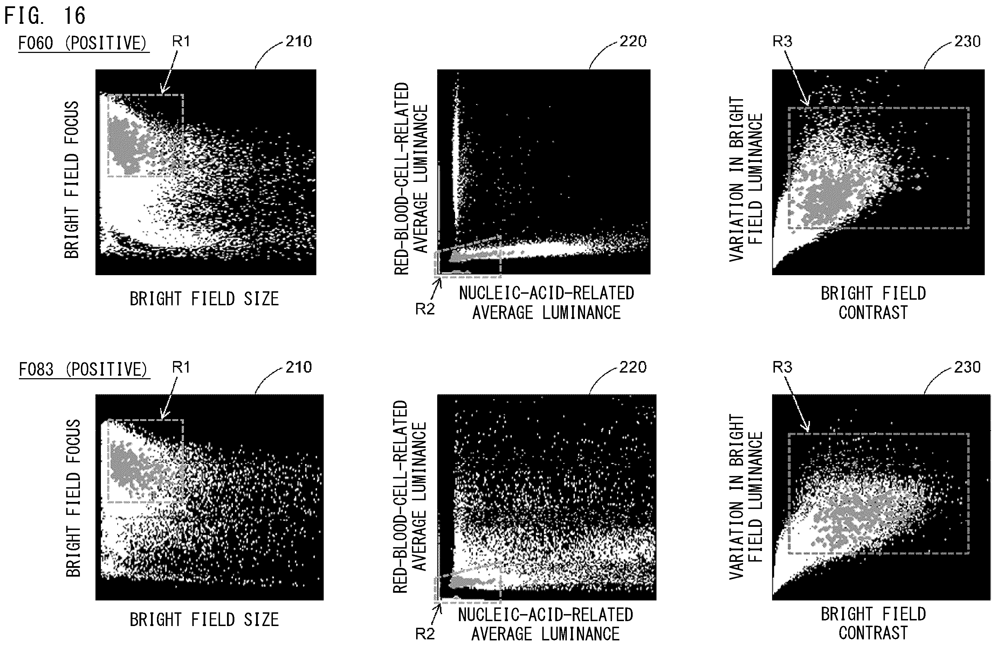

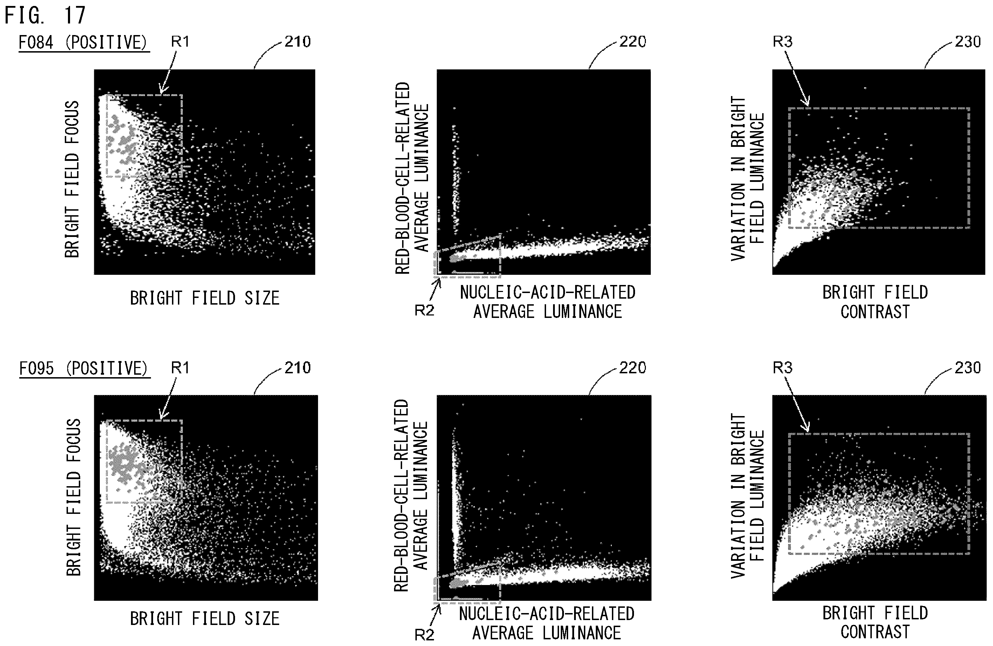

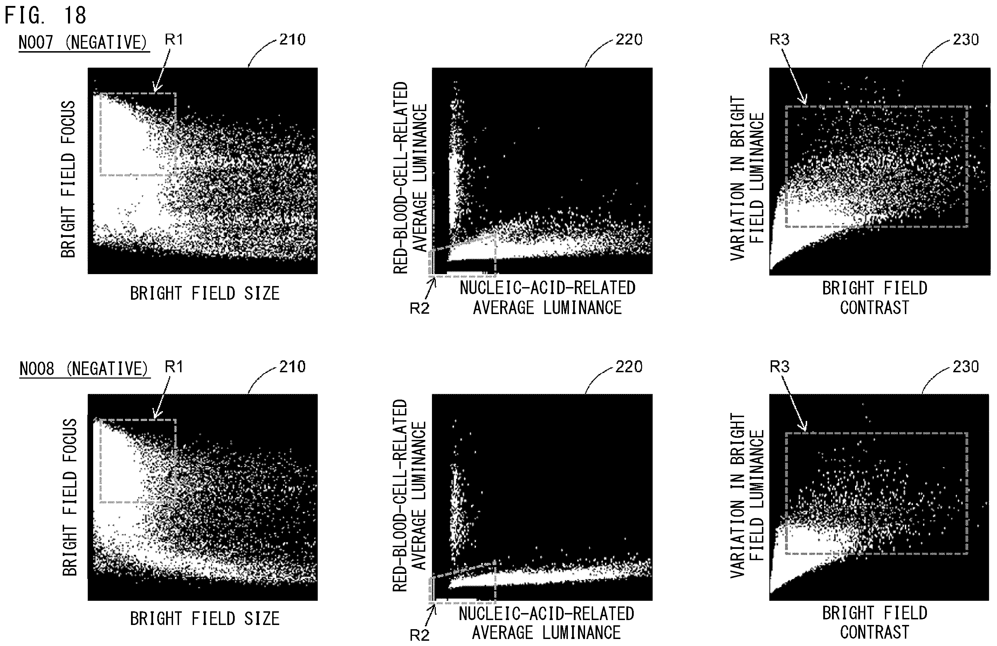

Next, an experiment in which the first to third extraction steps were performed on twelve subjects to extract mulberry bodies will be described with reference to FIG. 12 to FIG. 18.

In this experiment, UF-Fluorocell CR (manufactured by Sysmex Corporation) was used as a first fluorescent dye to stain a nucleic acid, Glycophorin A antibody (SC-53295; manufactured by SANTA CRUZ BIOTECHNOLOGY, INC.) was used as an antibody to specifically bind to a red blood cell, and MIX-n-STAIN CF594 Antibody Labeling Kits (92256; manufactured by Biotium, Inc.) were used as a second fluorescent dye to bind to the antibody. The wavelength λ11 for exciting the first fluorescent dye was 488 nm, and the wavelength 112 for exciting the second fluorescent dye was 592 nm.

FIG. 12 shows a table indicating the number of particles included in each of the ranges for the respective first to third extraction steps.

In this experiment, a urine specimen was collected from each of the twelve subjects, microscopy was performed on a portion of the collected urine specimen by a skilled laboratory technician, and the process of steps S1 to S6 in FIG. 3 was performed on another portion of the urine specimen.

The laboratory technician performed microscopy on a portion of each of the urine specimens. In the case of determining that mulberry bodies were contained, the laboratory technician regarded the urine specimen as being positive. Meanwhile, in the case of determining that mulberry bodies were not contained, the laboratory technician regarded the urine specimen as being negative. Urine specimens having specimen IDs of F005, F006, F032, F035, F047, F049, F060, F083, F084, and F095 were determined to be positive as a result of the microscopies. Urine specimens having specimen IDs of N007 and N008 were determined to be negative as a result of the microscopies.

Meanwhile, the process of steps S1 to S6 in FIG. 3 was performed on another portion of the same urine specimen, and, in each of the scattergram 210 obtained in step S4, the scattergram 220 obtained in step S5, and the scattergram 230 obtained in step S6, the number of particles and the number of mulberry bodies were counted, and the proportion of the mulberry bodies was calculated. Whether or not the particles were mulberry bodies was determined through visual checking, by the skilled laboratory technician, of images taken by the imaging unit 19 in step S3. The number indicated in the column "All" in the table is the number of particles included on the scattergram 210 obtained in step S4. The number indicated in the column "R1" in the table is the number of particles included in the range R1 on the scattergram 210 obtained in step S4. The number indicated in the column "R2" in the table is the number of particles included in the range R2 on the scattergram 220 obtained in step S5. The number indicated in the column "R3" in the table is the number of particles included in the range R3 on the scattergram 230 obtained in step S6. The proportion of mulberry bodies in each of the ranges is a proportion obtained by dividing the number of the mulberry bodies by the number of particles in the same range.

As shown in FIG. 12, the number of particles in each of the urine specimens significantly decreased as a result of performing the first to third extraction steps on all the particles imaged by the imaging unit 19. Meanwhile, the number of mulberry bodies in each of the urine specimens hardly decreased as a result of the first to third extraction steps. In other words, the number of mulberry bodies eventually extracted from the range R3 was only slightly smaller than the number of all the mulberry bodies imaged by the imaging unit 19. Consequently, the proportion of the mulberry bodies increased each time of extraction and eventually increased to as high as about 1% to 5%. Therefore, it is found that the mulberry bodies were able to be more accurately extracted from among all the particles imaged by the imaging unit 19.

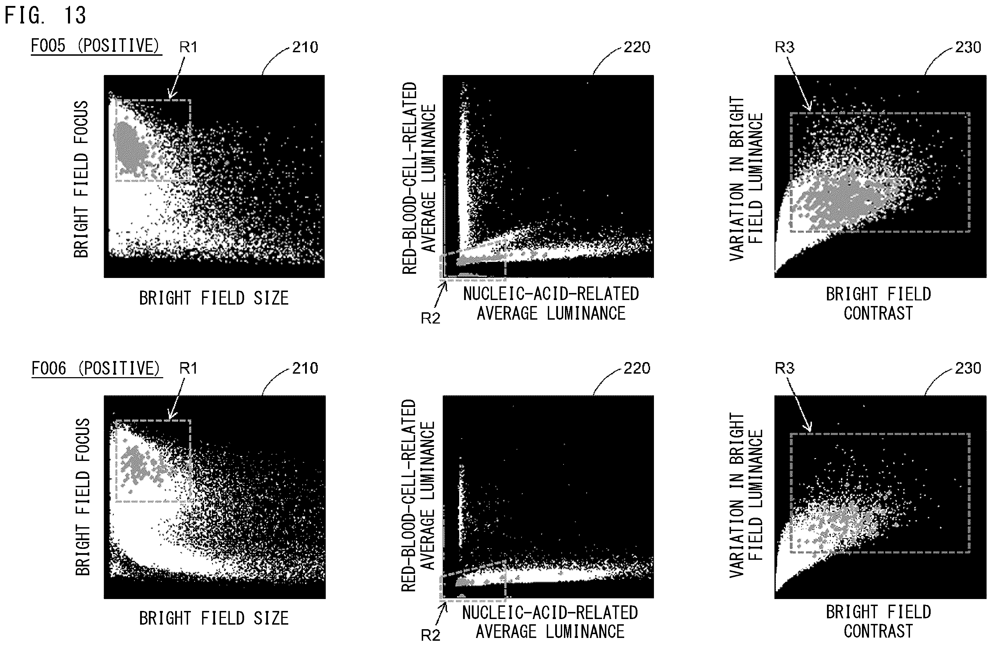

FIG. 13 to FIG. 18 show the scattergrams 210, 220, and 230 obtained in the first to third extraction steps with respect to the urine specimens shown in FIG. 12.

In FIG. 13 to FIG. 18, the plotted gray dots correspond to particles determined to be mulberry bodies through visual checking, by the skilled laboratory technician, of the images taken by the imaging unit 19 in step S3, and the plotted white dots correspond to particles other than the particles determined to be mulberry bodies.

Likewise, the plotted gray dots on the scattergrams 210, 220, and 230 in FIGS. 4, 6, and 9 also correspond to particles determined to be mulberry bodies through visual checking, by the skilled laboratory technician, of the images taken by the imaging unit 19 in step S3. Here, determination as to whether or not the particles are mulberry bodies through visual checking by the skilled laboratory technician, and use of different colors for the respective types of plotted dots according to the result of the determination, are intended to ascertain effects of this experiment and may be omitted.

The urine specimen having the specimen ID of F005 is identical to the urine specimen presented as an example in description made with reference to FIG. 4 to FIG. 11. Therefore, the scattergrams 210, 220, and 230 shown on the upper stage of FIG. 13 are identical to the scattergrams shown in FIGS. 4, 6, and 9. However, these scattergrams are shown again for convenience.

As is known with reference to the scattergrams 210, 220, and 230 regarding each of the urine specimens in FIG. 13 to FIG. 18, almost all of the plotted gray dots representing mulberry bodies fall within the ranges R1, R2, and R3 which are ranges for extraction performed through the first to third extraction steps. Thus, it is found that the mulberry bodies can be appropriately extracted from each of the urine specimens by using the ranges R1, R2, and R3 which are prestored in the storage 12.

Next, the process, of displaying images of the particles, performed in step S7 in FIG. 3 will be described with reference to FIG. 19 to FIG. 25. In step S7 in FIG. 3, the images of the particles extracted through the first to third extraction steps (the particles included in the range R3 on the scattergram 230 in FIG. 9) are displayed in the screen 300 described below, and histories of counting results of the mulberry bodies and information based on change in the counting results are displayed in a screen 400 described below.

FIG. 19 schematically shows a configuration of the screen 300 in which the images of the particles are displayed.

The screen 300 includes: a specimen ID display region 301; a subject ID display region 302; and an image display region 310 in which the images of the particles are displayed. In addition, the screen 300 includes a bright field image display button 321, a first fluorescence image display button 322, a second fluorescence image display button 323, and a third fluorescence image display button 324 each for selecting a type of image to be displayed in the image display region 310. In addition, the screen 300 includes a numerical order button 331 and a size-based order button 332 each for setting an arrangement order for the images to be displayed in the image display region 310. In addition, the screen 300 includes: a counting result display region 341 in which the number of mulberry bodies selected in the image display region 310 is displayed; and a confirmation button 342 for confirming the counting result.

When, after inputting a specimen ID and a subject ID, an operator inputs an instruction to display the screen 300, the processing unit 11 causes the display unit 14 to display the screen 300 shown in FIG. 19.

When the operator operates any of the bright field image display button 321, the first fluorescence image display button 322, the second fluorescence image display button 323, and the third fluorescence image display button 324, the processing unit 11 causes images of the type corresponding to the operated button to be displayed in the image display region 310. In the image display region 310, images of particles included in the range R3 and obtained from the urine specimen indicated in the specimen ID display region 301 are displayed. The example shown in FIG. 19 indicates a state where the bright field image display button 321 among the four buttons each for selecting a type of image is operated. As a result, bright field images are displayed in the image display region 310.

When the operator operates either of the numerical order button 331 and the size-based order button 332, the processing unit 11 switches the order of the images displayed in the image display region 310. The images are assigned with respective serial numbers for the particles, and, when the numerical order button 331 is operated, the images are rearranged in the order of the serial numbers. When the size-based order button 332 is operated, the images are rearranged in the order of the sizes of the particles in the images on the basis of the bright field sizes obtained in step S4. The example shown in FIG. 19 indicates a state where the numerical order button 331 out of the two buttons each for setting an arrangement order is operated. Each of the numerical order button 331 and the size-based order button 332 causes, by being successively operated, the display order to be switched between the ascending order and the descending order.

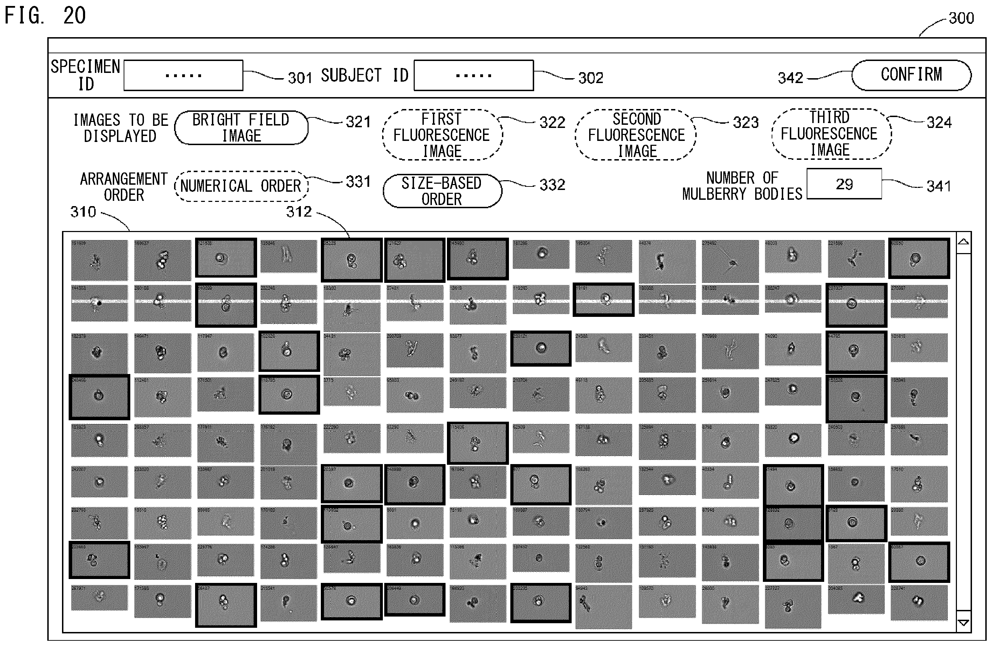

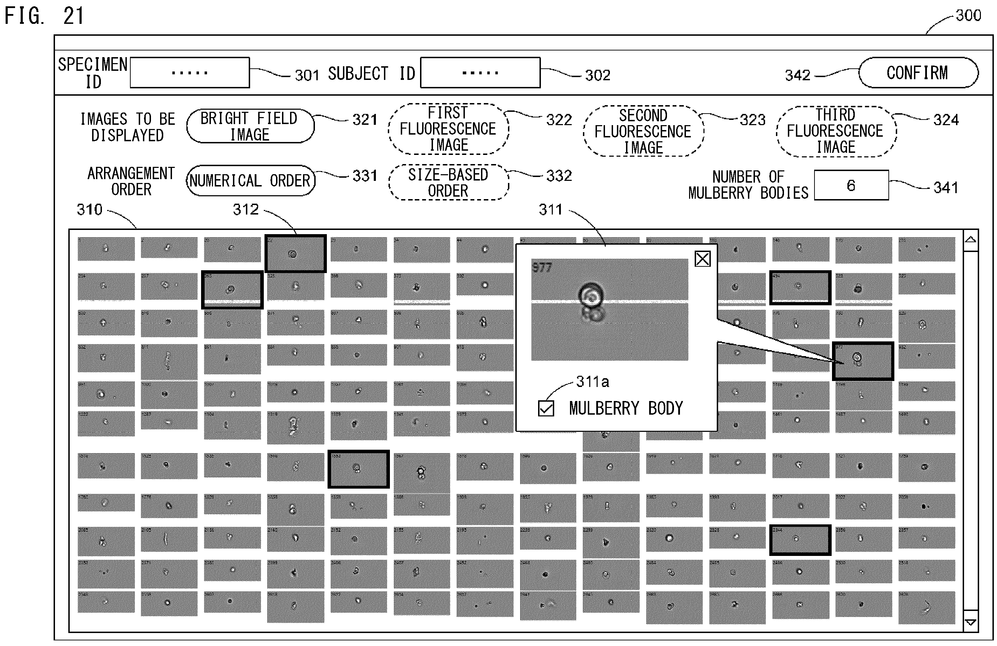

The example shown in FIG. 20 indicates a state where the size-based order button 332 out of the two buttons each for setting an arrangement order is operated. In FIG. 20, images of particles selected as mulberry bodies are enclosed by thick enclosing lines 312 as described later with reference to FIG. 21. Mulberry bodies have sizes approximately equal to one another, and thus arrangement of the images in the size-based order causes images corresponding to mulberry bodies to be arranged close to each other. Consequently, the operator can more accurately select the mulberry bodies.

When the screen 300 is displayed, the processing unit 11 may cause the images to be displayed in the image display region 310 in the numerical order or the size-based order. In addition, the screen 300 may have at least one of a button for rearranging the images in the order of bright field sizes, a button for rearranging the images in the order of bright field foci, a button for rearranging the images in the order of nucleic-acid-related average luminances, a button for rearranging the images in the order of red-blood-cell-related average luminances, a button for rearranging the images in the order of bright field contrasts, and a button for rearranging the images in the order of variations in bright field luminances.

The operator refers to each of the images in the image display region 310 and, when determining that the particle shown in the image might be a mulberry body, operates (e.g., clicks) the image. When the image in the image display region 310 is operated, the processing unit 11 causes an enlarged image region 311 to be displayed on the screen 300 in an overlapping manner.

FIG. 21 schematically shows a configuration of the screen 300 in which the enlarged image region 311 has been displayed.

In the enlarged image region 311, the image operated in the image display region 310 is displayed in an enlarged manner. The enlarged image region 311 includes a checkbox 311a. The operator further observes the enlarged image and, when determining that the particle shown in the image is a mulberry body, checks off the checkbox 311a.

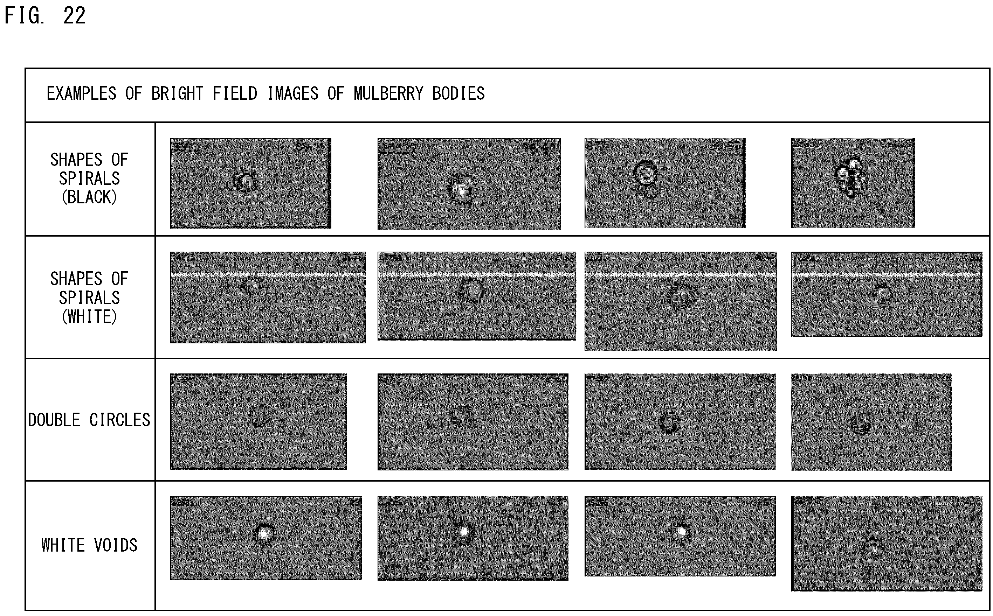

FIG. 22 shows exemplary bright field images of the particles determined to be mulberry bodies by the operator.

There are mulberry bodies having the shapes of black spirals as shown on the first stage, mulberry bodies having the shapes of white spirals as shown on the second stage, mulberry bodies having double circles as shown on the third stage, and mulberry bodies having white voids as shown on the fourth stage. After ascertaining these shapes of the mulberry bodies, the operator determines, with reference to the image display region 310 and the enlarged image region 311 shown in FIGS. 19 to 21, whether or not the particle shown in the image is a mulberry body. At this time, since much of the particles other than mulberry bodies have been excluded through the first to third extraction steps as described above, the operator can more accurately perform the task of continuously determining, with reference to each of the images shown in FIGS. 19 to 21, whether or not the particle is a mulberry body.

With reference back to FIG. 21, the processing unit 11 causes the image with the checkbox 311a being checked off to be stored in the storage 12 (see FIG. 1) such that a flag is associated with the image. Then, the processing unit 11 causes this image in the image display region 310 to be enclosed with a thick enclosing line 312. Enclosure of the image with the thick enclosing line 312 makes it easy to ascertain the image of the particle determined to be a mulberry body among the plurality of images in the image display region 310. In addition, association of the flag with the image having been checked off makes it possible to recheck, at a later date, the image of the particle determined to be a mulberry body.

Input of an instruction for the image is not limited to input performed via the checkbox 311a and may be input performed on the image via another operation (for example, a right click, a double click, or the like).

In addition, the processing unit 11 causes the number of the particles with the checkboxes 311a being checked off to be displayed in the counting result display region 341. Consequently, the operator can smoothly ascertain the number of the particles determined to be mulberry bodies (the number of the particles enclosed with the thick enclosing lines 312).

Further, when the operator operates the confirmation button 342, the processing unit 11 transmits, to the external host computer (see FIG. 1), a counting result including: the number of the particles determined to be mulberry bodies via the checkboxes 311a; the proportion of the number of the particles determined to be mulberry bodies to the number of the particles in the range R3; the specimen ID; the subject ID; and the present date and time. The counting result may be transmitted to the host computer and/or stored in the storage 12.

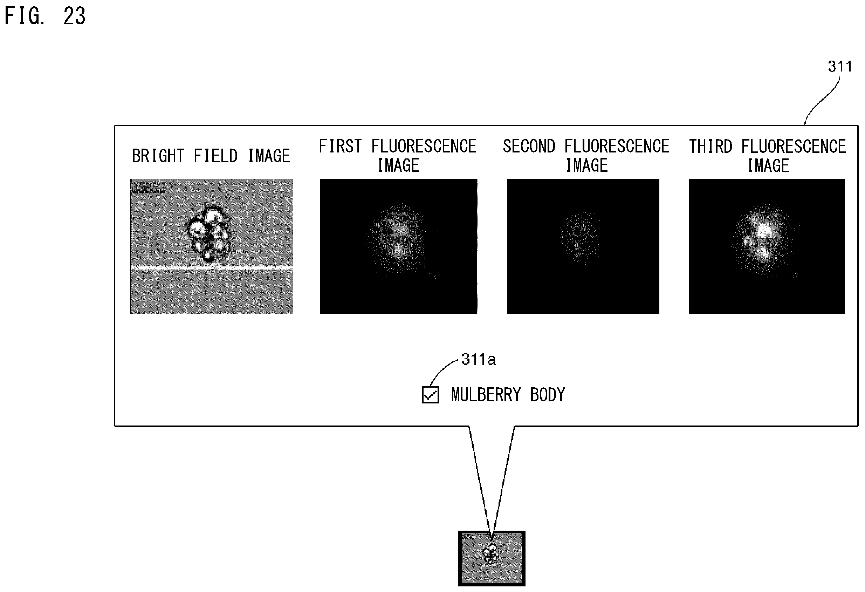

In FIG. 21, only an enlarged image of the image operated in the image display region 310 is displayed in the enlarged image region 311. However, without limitation thereto, all the images (the bright field image, the first fluorescence image, the second fluorescence image, and the third fluorescence image) corresponding to the particle in the image operated in the image display region 310 may be displayed as shown in FIG. 23. In this case, the operator can smoothly determine whether or not said particle is a mulberry body with reference to all the images. In this case as well, the third fluorescence image does not have to be displayed.

In addition, checkboxes regarding the shapes (the shapes of spirals, double circles, white voids, and the like) of mulberry bodies may be provided in the enlarged image region 311. In this case, the operator determines a shape of the mulberry body with reference to the image and checks off any of the checkboxes according to the shape of the mulberry body. Further, in the screen 300, a counting result is displayed for each of the shapes of mulberry bodies, and buttons for rearranging the images are provided for each of the shapes of mulberry bodies. Consequently, it becomes easy to check mulberry bodies having typical shapes (the shapes of spirals and double circles) and mulberry bodies having atypical shapes (the other shapes) in the image display region 310. Thus, for example, the effect of enzyme replacement therapy having been conducted on patients who suffer from Fabry disease can be confirmed.

In addition, the scattergrams 210, 220, and 230 shown in FIGS. 4, 6, and 9 may be displayed in the screen 300. In this case, when the operator operates an image in the image display region 310, a corresponding one of the particles plotted on each of the scattergrams may be marked, and, when the operator operates one of the particles plotted on any of the scattergrams, the corresponding image in the image display region 310 may be marked.

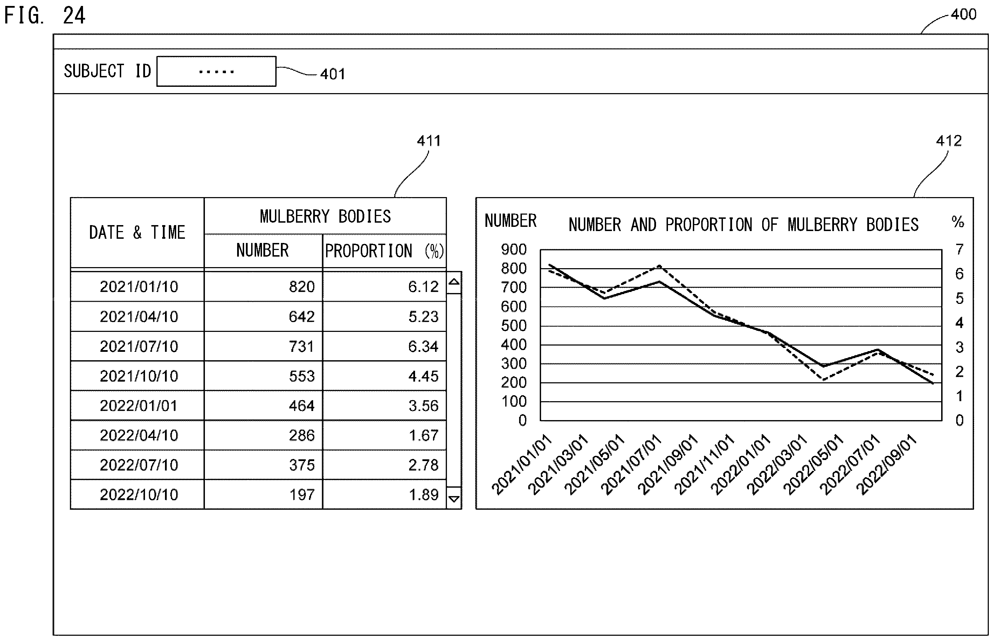

FIG. 24 schematically shows a configuration of the screen 400 in which histories of and change in counting results of mulberry bodies regarding a same subject are displayed.

The screen 400 includes a subject ID display region 401, a list display region 411, and a graph display region 412.

When, after inputting a subject ID, the operator inputs an instruction to display the screen 400, the processing unit 11 causes the display unit 14 to display the screen 400 shown in FIG. 24. At this time, the processing unit 11 inquires of the host computer (see FIG. 1) on the basis of the subject ID and receives a counting result corresponding to the subject ID from the host computer. When there are a plurality of counting results corresponding to one subject ID, the processing unit 11 receives the plurality of counting results as histories of counting results from the host computer. The processing unit 11 causes the list display region 411 and the graph display region 412 to be displayed on the basis of the received counting results.

Each row in the list display region 411 corresponds to one of the counting results. In the list display region 411, the numbers of particles determined to be mulberry bodies, and the proportions of the numbers of the particles determined to be mulberry bodies to the numbers of particles in the range R3, are displayed. In the graph display region 412, a graph based on the data in the list display region 411 is displayed. The graph in the graph display region 412 is information based on change in the counting results.

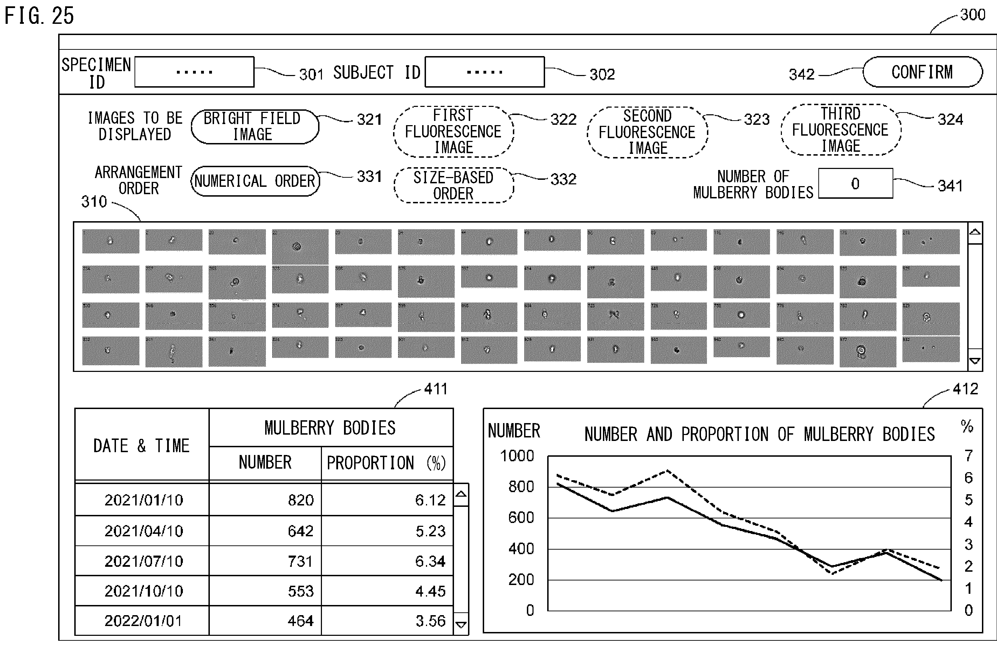

The list display region 411 and the graph display region 412 shown in FIG. 24 are displayed in the screen 400 different from the screen 300 shown in FIGS. 19 to 21 but may be displayed together in the screen 300 as shown in FIG. 25.

### <Advantageous Effects of Particle Detection Method, Particle Detection Apparatus, and Program according to Embodiment>

In step S1 in FIG. 3, the processing unit 11 drives the sample preparation unit 18 to prepare a sample containing a urine specimen collected from a subject, a first fluorescent dye that stains a nucleic acid, an antibody that specifically binds to a red blood cell, and a second fluorescent dye that binds to the antibody that specifically binds to the red blood cell. In step S3, the processing unit 11 causes each of a plurality of particles in the sample to be imaged to obtain a fluorescence image. In step S5, the processing unit 11 extracts a particle regarding which information obtained from the corresponding fluorescence image is included in the range R2 (see FIG. 6) corresponding to mulberry bodies.

Through this process, the particle regarding which the information obtained from the corresponding fluorescence image is included in the range R2 corresponding to mulberry bodies is extracted, whereby mulberry bodies can be more accurately extracted. In particular, mulberry bodies can be more accurately extracted with exclusion of red blood cells and fungi that are morphologically similar to mulberry bodies.

The information obtained from the corresponding fluorescence image includes a value related to an amount of a first fluorescence generated from the particle by the first fluorescent dye, and a value related to an amount of a second fluorescence generated from the particle by the second fluorescent dye.

With this configuration, red blood cells, tubular epithelial cells, urothelial cells, white blood cells, fungi, and the like can be excluded.

The fluorescence image includes a first fluorescence image obtained by imaging the fluorescence generated by the first fluorescent dye, and a second fluorescence image obtained by imaging the fluorescence generated by the second fluorescent dye.

With this configuration, the value related to the amount of the first fluorescence and the value related to the amount of the second fluorescence can be smoothly obtained by individually imaging the fluorescence generated by the first fluorescent dye and the fluorescence generated by the second fluorescent dye, respectively.

The extracting of the particle includes a second extraction step (step S5 in FIG. 3) of extracting a particle regarding which the value related to the amount of the first fluorescence and the value related to the amount of the second fluorescence are included in the range R2 (i.e., satisfy a predetermined condition).

With this configuration, use of the predetermined condition makes it possible to extract mulberry bodies, whereby extraction can be smoothly performed.

In step S3 in FIG. 3, the processing unit 11 causes each of the plurality of particles in the sample to be imaged to further obtain a bright field image. The extracting of the particle includes the first extraction step (step S4), the second extraction step (step S5), and the third extraction step (step S6), each of the steps being a step of extracting a particle regarding which the information obtained from the corresponding fluorescence image and information obtained from the corresponding bright field image are included in the corresponding one of the ranges R1, R2, and R3 (see FIGS. 4, 6, and 9) corresponding to the mulberry bodies.

With this configuration, use of the bright field image makes it possible to further accurately extract mulberry bodies.

The information obtained from the corresponding bright field image includes a value related to a size of the particle based on the bright field image, and a value related to a focus state of the particle based on the bright field image.

With this configuration, the value related to the size of the particle makes it possible to exclude urine specimen particles (a single bacterium, a urothelial cell having a large size, a squamous epithelial cell, and the like) other than mulberry bodies. In addition, the value related to the focus state makes it possible to exclude images of particles that are not suitable for visual checking.

The information obtained from the corresponding bright field image includes a value related to a contrast of the particle based on the bright field image, and a value related to a variation in a luminance of the particle based on the bright field image.

With this configuration, particles indicated with only brightness/darkness, debris, particles that are not focused, aggregates of small particles, and the like can be excluded.

The extracting of the particle includes the first extraction step (step S4), the second extraction step (step S5), and the third extraction step (step S6) as shown in FIG. 3. In the first extraction step, the processing unit 11 extracts a particle regarding which a value related to a size of the particle based on the bright field image and a value related to a focus state of the particle based on the bright field image are included in the range R1 in FIG. 4 (i.e., satisfy a first condition). In the second extraction step, the processing unit 11 obtains, from the first fluorescence image, a value related to an amount of a first fluorescence generated from the particle by the first fluorescent dye, obtains, from the second fluorescence image, a value related to an amount of a second fluorescence generated from the particle by the second fluorescent dye, and extracts a particle regarding which the value related to the amount of the first fluorescence and the value related to the amount of the second fluorescence are included in the range R2 in FIG. 6 (i.e., satisfy a second condition). In the third extraction step, the processing unit 11 extracts a particle regarding which a value related to a contrast of the particle based on the bright field image and a value related to a variation in a luminance of the particle based on the bright field image are included in the range R3 in FIG. 9 (i.e., satisfy a third condition).

Through the first to third extraction steps, mulberry bodies can be more accurately extracted.

The range R2 corresponding to the mulberry bodies is a range that overlaps with neither the range R41 corresponding to red blood cells nor the range R42 corresponding to fungi.

With this configuration, mulberry bodies can be more accurately extracted with exclusion of red blood cells and fungi that are morphologically similar to mulberry bodies.

In step S7 in FIG. 3, the processing unit 11 causes an image of the particle that has been extracted and that is included in the range corresponding to the mulberry bodies (for example, the range R3 in FIG. 9) to be displayed in the screen 300 shown in FIGS. 19 to 21.

This step enables the operator to, with reference to the image of the particle included in the range corresponding to the mulberry bodies, smoothly check the particle having a high probability of being a mulberry body.

The processing unit 11 causes each of the plurality of particles in the sample to be imaged to obtain a bright field image in step S3 in FIG. 3 and causes the bright field image of the extracted particle to be displayed in the image display region 310 shown in FIGS. 19 to 21 in step S7 in FIG. 3.

Features of mulberry bodies are likely to appear in bright field images. Therefore, these steps enable the operator to, with reference to the bright field images, smoothly check the particles having a high probability of being mulberry bodies.

In step S7 in FIG. 3, the processing unit 11 causes the images of the extracted particles to be listed in the image display region 310 shown in FIGS. 19 to 21.

Through this step, the images of the particles are listed, whereby it becomes easy for the operator to sequentially check the images of the plurality of particles.

In step S7 in FIG. 3, the processing unit 11 causes the images of the extracted particles to be listed in an order of sizes of the particles in the images in the image display region 310 as shown in FIG. 20.

Rearrangement in the order of the sizes of the particles makes it easy to separately display mulberry bodies and the other particles in the image display region 310. Consequently, the operator can more accurately check mulberry bodies.

In step S7 in FIG. 3, the processing unit 11 receives, via the size-based order button 332 in FIGS. 19 to 21, input of an instruction to arrange the images of the extracted particles in an order of sizes of the particles, and rearranges the listed images of the particles according to the received input of the instruction.

This step enables the operator to smoothly rearrange the listed particles.

In step S7 in FIG. 3, the processing unit 11 receives input of an instruction to specify any one of the listed images of the particles, and causes the image of the particle specified through the input of the instruction to be displayed in an enlarged manner as shown in the enlarged image region 311 in FIG. 21.

This step makes it easy for the operator to see the details of the particle, whereby the operator can more accurately check whether or not said particle is a mulberry body.

In step S7 in FIG. 3, the processing unit 11 receives, via the checkboxes 311a in FIG. 21, input of an instruction to specify images of the mulberry bodies among the listed images of the particles, and causes a counting result obtained by counting the images of the particles specified through the input of the instruction to be displayed in the counting result display region 341 as shown in FIG. 21.

Through this step, the number of the particles specified as mulberry bodies by the operator can be used as information that is effective in pathologic diagnoses such as determination as to whether or not the subject has developed Fabry disease.

The processing unit 11 stores histories of the counting results regarding a same subject such as ones shown in the list display region 411 in FIG. 24.

Since such histories of the counting results are displayed, the operator can check, as appropriate, transition of the state of mulberry bodies in said subject.

As shown in the graph display region 412 in FIG. 24, the processing unit 11 causes information based on change in the counting results to be displayed on the basis of the histories of the counting results.

This step enables the operator to smoothly check change in the number of mulberry bodies in said subject. Therefore, it is possible to more accurately conduct pathologic diagnoses such as: determination as to whether or not the subject has developed Fabry disease; and determination of the efficacy of a medication administered to the subject.

As shown in the screen 300 in FIG. 25, the processing unit 11 causes the information based on the change in the counting results shown in the graph display region 412 to be displayed together with the listed images of the particles shown in the image display region 310.

This step makes it possible to simultaneously refer to the listed images and the change in the counting results. For example, whether the medication administered to the subject or the like has been effective can be checked.

In step S7 in FIG. 3, the processing unit 11 receives, via the checkbox 311a in FIG. 21, input of an instruction to specify an image of any of the mulberry bodies among the listed images of the particles, and stores the image of the particle specified through the input of the instruction such that a flag is associated with the image.

This step enables the image of the particle determined to be a mulberry body by the operator to be subsequently displayed together with a marking. Consequently, for example, the result of determination by the operator can be smoothly checked by a doctor at a later date. Therefore, a diagnosis can be more accurately conducted.

As shown in FIG. 2, the imaging unit 19 includes the flow cell 110 through which the sample is caused to flow, and the imaging unit 19 images the plurality of particles in the sample flowing through the flow cell 110.

With this configuration, images of a large number of particles can be efficiently obtained.

### <First Modification of Particle Detection Apparatus, Method, and Program>

In the above embodiment, both the first extraction step and the third extraction step are executed. However, one of the first extraction step and the third extraction step may be omitted, or both the first extraction step and the third extraction step may be omitted.

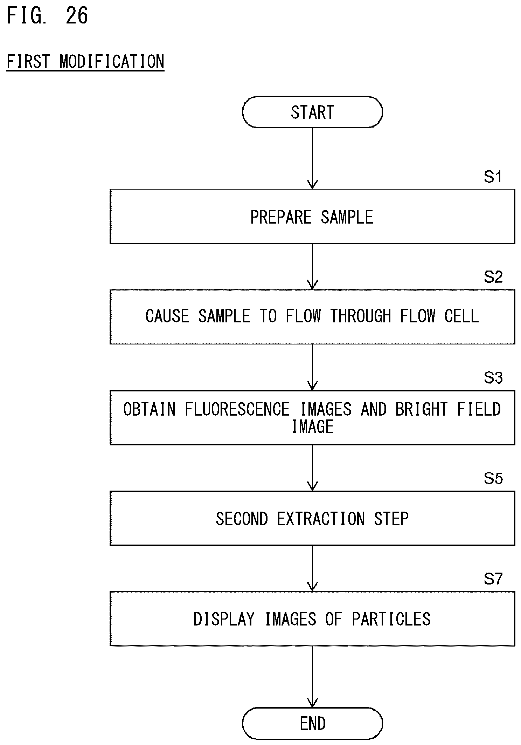

FIG. 26 is a flowchart showing a process regarding a particle detection method to be performed by the processing unit 11 in a case where: the sample prepared in step S1 does not contain the Gb3 antibody labeled with the third fluorescent dye; and only the second extraction step is performed as an extraction step.

In comparison with the process according to the embodiment in FIG. 3, the process in FIG. 26 includes neither the first extraction step as step S4 nor the third extraction step as step S6.

In this case, the processing unit 11 obtains bright field images, first fluorescence images, and second fluorescence images in the second extraction step as step S5. In addition, the processing unit 11 plots all the particles imaged by the imaging unit 19 onto the scattergram 220 in FIG. 6 and extracts particles in the range R2 on the scattergram 220. Then, in step S7, the processing unit 11 causes images of the particles extracted through the second extraction step as step S5 to be displayed in the screen 300.

In this manner, even in the case of performing only the second extraction step as an extraction step, particles are extracted from within the range R2 which is defined by: the values related to the amounts of the first fluorescence generated from particles by the first fluorescent dye; and the values related to the amounts of the second fluorescence generated from the particles by the second fluorescent dye. In the range R2, as described above, mulberry bodies are included, and red blood cells, tubular epithelial cells, urothelial cells, white blood cells, and fungi are hardly included. Therefore, in the process shown in FIG. 26 as well, mulberry bodies can be more accurately extracted with exclusion of these particles other than mulberry bodies.

### <Second Modification of Particle Detection Apparatus, Method, and Program>

In the above embodiment, both the values related to the sizes of the particles based on the bright field images and the values related to the focus states of the particles based on the bright field images are used in the first extraction step as shown in FIG. 4. However, without limitation thereto, it is possible to use only the values related to the sizes or only the values related to the focus states. A first extraction step in a particle detection method according to the present second modification will be described below by using, as an example, images obtained from the urine specimen having the specimen ID of "F005" shown in FIG. 12.

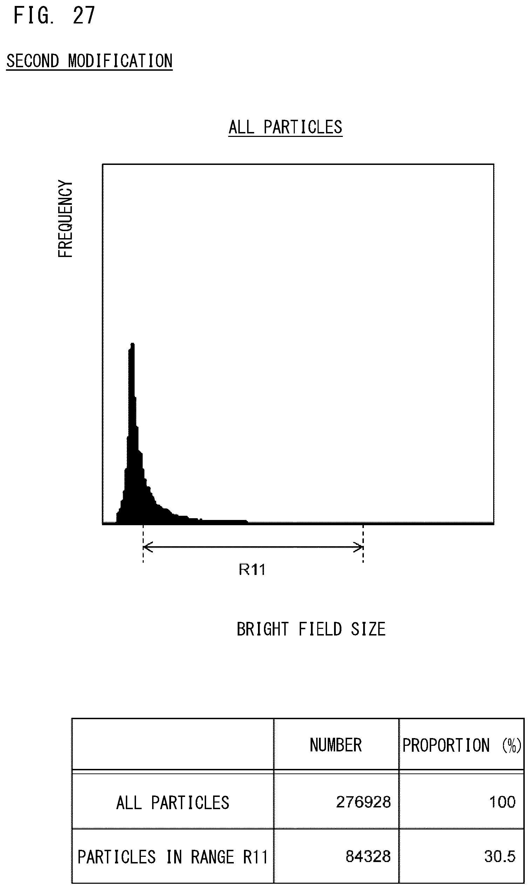

FIG. 27 shows an example in which the first extraction step is performed by using only the values related to the sizes of the particles based on the bright field images.

The graph in FIG. 27 is a graph based on all the particles imaged by the imaging unit 19. The graph in FIG. 27 has a horizontal axis indicating bright field size and a vertical axis indicating frequency. A range R11 is identical to the range of bright field sizes (the range in the horizontal axis direction) in the range R1 in FIG. 4.

In the first extraction step in this case, particles included in the range R11 (particles satisfying the first condition) are extracted. Consequently, in the case of the urine specimen having the specimen ID of "F005", 84328 particles in the range R11 are extracted from among all the 276928 particles imaged by the imaging unit 19. That is, 30.5% of all the particles are extracted, and 69.5% of all the particles are excluded.

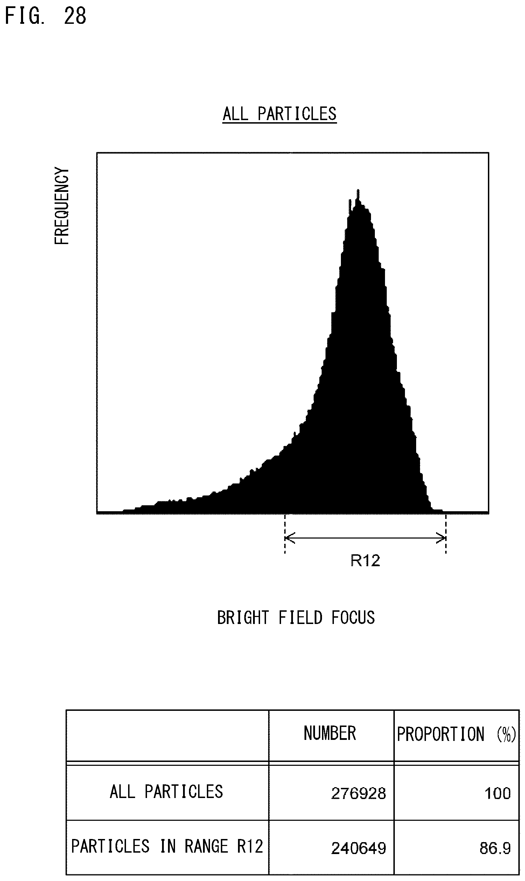

FIG. 28 shows an example in which the first extraction step is performed by using only the values related to the focus states of the particles based on the bright field images.

The graph in FIG. 28 has a horizontal axis indicating bright field focus and a vertical axis indicating frequency. The graph in FIG. 28 is a graph based on all the particles imaged by the imaging unit 19. A range R12 is identical to the range of bright field foci (the range in the vertical axis direction) in the range R1 in FIG. 4.

In the first extraction step in this case, particles included in the range R12 (particles satisfying the first condition) are extracted. Consequently, in the case of the urine specimen having the specimen ID of "F005", 240649 particles in the range R12 are extracted from among all the 276928 particles imaged by the imaging unit 19. That is, 86.9% of all the particles are extracted, and 13.1% of all the particles are excluded. Since the ranges R11 and R12 are ranges including at least the particles in the range R1, the ranges R11 and R12 include mulberry bodies, the number of which is equal to or larger than that in the range R1.

As described above, even when only either of the values related to the sizes of the particles based on the bright field images and the values related to the focus states of the particles based on the bright field images are used in the first extraction step, particles having a low probability of being mulberry bodies can be significantly excluded, and mulberry bodies can be more accurately extracted.

In addition, as is known also from the above example in which the specimen ID is "F005", the proportion (69.5%) of particles excluded through the first extraction step in which only the values related to the sizes are used is higher than the proportion (13.1%) of particles excluded through the first extraction step in which only the values related to the focus states are used. Therefore, at least the bright field sizes (the values related to the sizes of the particles) are preferably used in the first extraction step.

In addition, the order of execution of the first extraction step, the second extraction step, and the third extraction step is not limited to the order described in the above embodiment. However, in the case of using at least the values related to the sizes in the first extraction step, the first extraction step is preferably executed prior to the second extraction step and the third extraction step. In this case, in a state where the number of the particles is significantly decreased through the first extraction step, the process can be advanced to the subsequent steps, whereby the amount of computation by the processing unit 11 in the subsequent steps can be decreased.

### <Third Modification of Particle Detection Apparatus, Method, and Program>

In the above embodiment, both the values related to the contrasts of the particles based on the bright field images and the values related to the variations in the luminances of the particles based on the bright field images are used in the third extraction step as shown in FIG. 9. However, without limitation thereto, it is possible to use only the values related to the contrasts or only the values related to the variations in the luminances. A third extraction step in a particle detection method according to the present third modification will be described below by using, as an example, the images obtained from the urine specimen having the specimen ID of "F005" shown in FIG. 12.

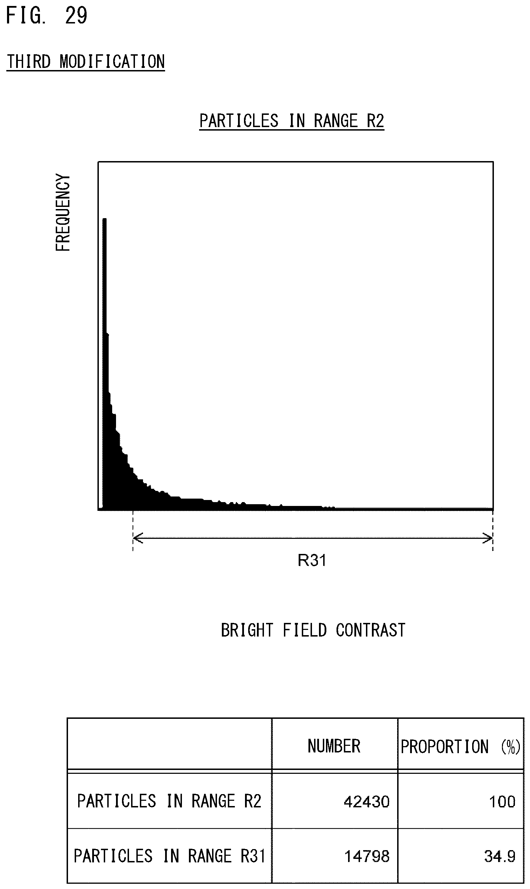

FIG. 29 shows an example in which the third extraction step is performed by using only the values related to the contrasts of the particles based on the bright field images.

The graph in FIG. 29 is a graph based on the particles extracted through the second extraction step (the particles extracted from within the range R2 in FIG. 6). The graph in FIG. 29 has a horizontal axis indicating bright field contrast and a vertical axis indicating frequency. A range R31 is identical to the range of bright field contrasts (the range in the horizontal axis direction) in the range R3 in FIG. 9.

In the third extraction step in this case, particles included in the range R31 are extracted. Consequently, in the case of the urine specimen having the specimen ID of "F005", 14798 particles in the range R31 are extracted from among 42430 particles extracted from within the range R2. That is, 34.9% of the particles in the range R2 are extracted, and 65.1% of the particles in the range R2 are excluded.

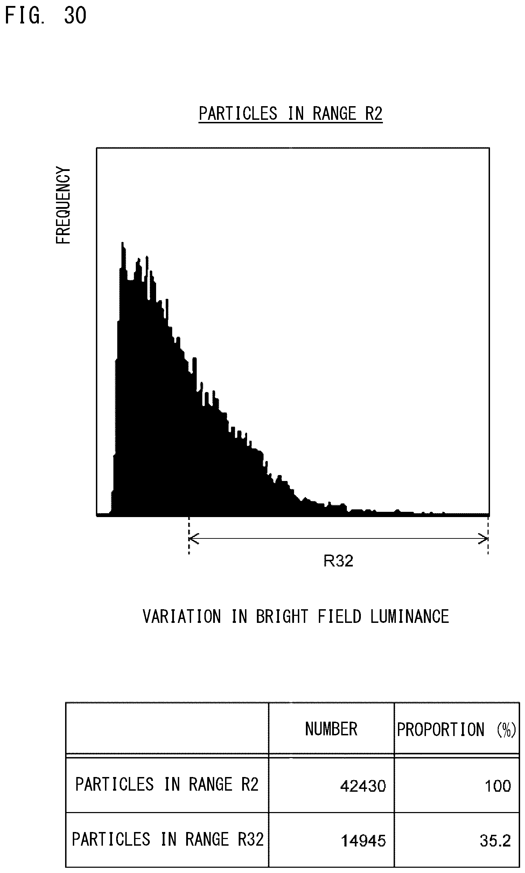

FIG. 30 shows an example in which the third extraction step is performed by using only the values related to the variations in the luminances of the particles.

The graph in FIG. 30 is a graph based on the particles extracted through the second extraction step (the particles extracted from within the range R2 in FIG. 6). The graph in FIG. 30 has a horizontal axis indicating variation in bright field luminance and a vertical axis indicating frequency. A range R32 is identical to the range of variations in bright field luminances (the range in the vertical axis direction) in the range R3 in FIG. 9.

In the third extraction step in this case, particles included in the range R32 are extracted. Consequently, in the case of the urine specimen having the specimen ID of "F005", 14945 particles in the range R32 are extracted from among 42430 particles extracted from within the range R2. That is, 35.2% of the particles in the range R2 are extracted, and 64.8% of the particles in the range R2 are excluded. Since the ranges R31 and R32 are ranges including at least the particles in the range R3, the ranges R31 and R32 include mulberry bodies, the number of which is equal to or larger than that in the range R3.

As described above, even when either of the values related to the contrasts of the particles based on the bright field images and the values related to the variations in the luminances of the particles based on the bright field images are used in the third extraction step, particles having a low probability of being mulberry bodies can be significantly excluded, and mulberry bodies can be more accurately extracted.

### <Other Modifications>

In the above embodiment, the first extraction step, the second extraction step, and the third extraction step are executed in this order as shown in FIG. 3. However, the order of execution of the first extraction step, the second extraction step, and the third extraction step is not limited thereto. For example, the second extraction step, the first extraction step, and the third extraction step may be executed in this order. When the order of execution of the three extraction steps is switched, the first extraction step, the second extraction step, and the third extraction step are such that extraction is executed with respect to a group of particles having been extracted through a previously-executed one of the extraction steps. Even when the order of execution of the three extraction steps is switched, the particles that are eventually extracted are the same as those in the above embodiment.

However, as is known with reference to the numbers of the particles in FIG. 12, the number of particles that can be excluded through the first extraction step is much larger than the number of particles that can be excluded through the other extraction steps. Therefore, when the first extraction step among the three extraction steps is performed first, the process can be advanced to the subsequent steps in a state where the number of particles is decreased, whereby the amount of computation by the processing unit 11 in the subsequent steps can be decreased.

In the above embodiment, the images of the particles are displayed in step S7 in FIG. 3. However, processing in step S7 may be omitted. In this case, for example, the particles extracted through the first to third extraction steps may be subjected to image analysis by the processing unit 11 or another device so that determination is performed as to the possibility that the particles are mulberry bodies.

In the above embodiment, each nucleic-acid-related average luminance (the value related to the amount of the first fluorescence generated by the first fluorescent dye) is obtained from the corresponding first fluorescence image, and each red-blood-cell-related average luminance (the value related to the amount of the second fluorescence generated by the second fluorescent dye) is obtained from the corresponding second fluorescence image. However, without limitation thereto, both the fluorescence generated by the first fluorescent dye and the fluorescence generated by the second fluorescent dye may be guided onto the same light reception surface of the camera 154, and the camera 154 may image these two fluorescences to generate one fluorescence image that is rendered in colors. In this case, for example, the processing unit 11 extracts, from the one fluorescence image having been generated, both a nucleic-acid-related average luminance and a red-blood-cell-related average luminance through image processing.

In the above embodiment, as shown in FIG. 2, the imaging unit 19 includes the flow cell 110 and images the plurality of particles in the sample flowing through the flow cell 110. However, without limitation thereto, the imaging unit 19 may be implemented by a microscope. In this case, the microscope images the plurality of particles in the sample on a glass slide and generates, for each of the particles, at least a fluorescence image and preferably a first fluorescence image, a second fluorescence image, and a bright field image. The processing unit 11 uses the images obtained by the microscope to more accurately extract mulberry bodies in the same manner as in the above embodiment.

In the above embodiment, the ranges R1 to R3 are fixed regions. However, at least one of the ranges R1 to R3 may be a variable region. For example, at least one of the ranges R1 to R3 may be a range that varies according to the state of distribution of the particles on the corresponding scattergram.

Various modifications can be made as appropriate to the embodiment of the present disclosure, without departing from the scope of the invention as defined by the claims.

## Claims

1. A particle detection method comprising:
preparing a sample containing a urine specimen collected from a subject, a first fluorescent dye that stains a nucleic acid, an antibody that specifically binds to a red blood cell, and a second fluorescent dye that binds to the antibody (S1);
imaging each of a plurality of particles in the sample, to obtain fluorescence images (S3); and
extracting a particle regarding which information obtained from the corresponding fluorescence image is included in a range (R2) corresponding to mulberry bodies (S5).

2. The particle detection method of claim 1, wherein
the information obtained from the corresponding fluorescence image includes
a value related to an amount of a first fluorescence generated from the particle by the first fluorescent dye, and
a value related to an amount of a second fluorescence generated from the particle by the second fluorescent dye.

3. The particle detection method of claim 2, wherein
the fluorescence image includes
a first fluorescence image obtained by imaging the fluorescence generated by the first fluorescent dye, and
a second fluorescence image obtained by imaging the fluorescence generated by the second fluorescent dye.

4. The particle detection method of claim 2, wherein
the extracting of the particle includes extracting a particle regarding which the value related to the amount of the first fluorescence and the value related to the amount of the second fluorescence satisfy a predetermined condition.

5. The particle detection method of claim 1, further comprising
imaging each of the plurality of particles in the sample, to obtain bright field images, wherein
the extracting of the particle includes extracting a particle regarding which the information obtained from the corresponding fluorescence image and information obtained from the corresponding bright field image are included in the range corresponding to the mulberry bodies.

6. The particle detection method of claim 5, wherein
the information obtained from the corresponding bright field image includes at least one of
a value related to a size of the particle based on the bright field image, and
a value related to a focus state of the particle based on the bright field image.

7. The particle detection method of claim 5 or 6, wherein
the information obtained from the corresponding bright field image includes at least one of
a value related to a contrast of the particle based on the bright field image, and
a value related to a variation in a luminance of the particle based on the bright field image.

8. The particle detection method of claim 5, wherein
the fluorescence image includes
a first fluorescence image obtained by imaging a fluorescence generated by the first fluorescent dye, and
a second fluorescence image obtained by imaging a fluorescence generated by the second fluorescent dye, and
the extracting of the particle includes
a first extraction step (S4) of extracting a particle regarding which at least one of a value related to a size of the particle based on the bright field image and a value related to a focus state of the particle based on the bright field image satisfies a first condition,
a second extraction step (S5) of
obtaining, from the first fluorescence image, a value related to an amount of a first fluorescence generated from the particle by the first fluorescent dye,
obtaining, from the second fluorescence image, a value related to an amount of a second fluorescence generated from the particle by the second fluorescent dye, and
extracting a particle regarding which the value related to the amount of the first fluorescence and the value related to the amount of the second fluorescence satisfy a second condition, and
a third extraction step (S6) of extracting a particle regarding which at least one of a value related to a contrast of the particle based on the bright field image and a value related to a variation in a luminance of the particle based on the bright field image satisfies a third condition.

9. The particle detection method of claim 8, wherein
the first extraction step (S4), the second extraction step (S5), and the third extraction step (S6) are such that extraction is executed with respect to a group of particles having been extracted through a previously-executed one of the extraction steps.

10. The particle detection method of claim 9, wherein
the first extraction step (S4) includes extracting a particle regarding which at least the value related to the size of the particle satisfies the first condition, and
the first extraction step (S4) is executed prior to the second extraction step (S5) and the third extraction step (S6).

11. The particle detection method of claim 1, wherein
the range corresponding to the mulberry bodies is a range that overlaps with neither a range corresponding to red blood cells nor a range corresponding to fungi.

12. The particle detection method of claim 1, further comprising
displaying an image of the particle that has been extracted and that is included in the range corresponding to the mulberry bodies (S7).

13. The particle detection method of claim 12, further comprising
imaging each of the plurality of particles in the sample, to obtain bright field images, wherein
the displaying of the image of the particle includes displaying the bright field image of the extracted particle.

14. A particle detection apparatus (1) comprising
a sample preparation unit (18) configured to prepare a sample containing a urine specimen collected from a subject, a first fluorescent dye that stains a nucleic acid, an antibody that specifically binds to a red blood cell, and a second fluorescent dye that binds to the antibody;
an imaging unit (19) configured to image each of a plurality of particles in the sample, to obtain fluorescence images; and
a processing unit (11) configured to extract a particle regarding which information obtained from the corresponding fluorescence image is included in a range (R2) corresponding to mulberry bodies.

15. The particle detection apparatus of claim 14, wherein
the imaging unit (19) includes a flow cell (110) through which the sample is caused to flow, and
the imaging unit (19) images the plurality of particles in the sample flowing through the flow cell (110).

16. A program (12a) configured to cause a computer to execute a process of detecting a particle in a urine specimen collected from a subject, the process comprising
extracting a particle regarding which information obtained from a corresponding fluorescence image is included in a range (R2) corresponding to mulberry bodies, the corresponding fluorescence image being obtained by imaging each of a plurality of particles in a sample containing the urine specimen, a first fluorescent dye that stains a nucleic acid, an antibody that specifically binds to a red blood cell, and a second fluorescent dye that binds to the antibody.

## Patentansprüche

1. Partikeldetektionsverfahren, umfassend:
Vorbereiten einer Probe, die eine von einem Subjekt gesammelte Urinprobe, einen ersten Fluoreszenzfarbstoff, der eine Nukleinsäure färbt, einen Antikörper, der spezifisch an ein rotes Blutkörperchen bindet, und einen zweiten Fluoreszenzfarbstoff, der an den Antikörper bindet, enthält (S1);
Aufnehmen eines Bildes von jedem einer Vielzahl von Partikeln in der Probe, um Fluoreszenzbilder zu erhalten (S3); und
Extrahieren eines Partikels, bezüglich dessen Informationen, die aus dem entsprechenden Fluoreszenzbild erhalten wurden, in einem Bereich (R2) enthalten sind, der Maulbeerkörpern entspricht (S5).

2. Partikeldetektionsverfahren nach Anspruch 1, wobei
die aus dem entsprechenden Fluoreszenzbild erhaltenen Informationen einen Wert, der sich auf eine Menge einer ersten Fluoreszenz bezieht, die durch den ersten Fluoreszenzfarbstoff von dem Partikel erzeugt wird, und einen Wert, der sich auf eine Menge einer zweiten Fluoreszenz bezieht, die durch den zweiten Fluoreszenzfarbstoff von dem Partikel erzeugt wird, beinhalten.

3. Partikeldetektionsverfahren nach Anspruch 2, wobei
das Fluoreszenzbild ein erstes Fluoreszenzbild, das durch Aufnehmen der durch den ersten Fluoreszenzfarbstoff erzeugten Fluoreszenz erhalten wird, und ein zweites Fluoreszenzbild, das durch Aufnehmen der durch den zweiten Fluoreszenzfarbstoff erzeugten Fluoreszenz erhalten wird, beinhaltet.

4. Partikeldetektionsverfahren nach Anspruch 2, wobei
das Extrahieren des Partikels das Extrahieren eines Partikels beinhaltet, bezüglich dessen der Wert, der sich auf die Menge der ersten Fluoreszenz bezieht, und der Wert, der sich auf die Menge der zweiten Fluoreszenz bezieht, eine vorbestimmte Bedingung erfüllen.

5. Partikeldetektionsverfahren nach Anspruch 1, ferner umfassend Aufnehmen eines Bildes von jedem der Vielzahl von Partikeln in der Probe, um Hellfeld-Bilder zu erhalten, wobei
das Extrahieren des Partikels das Extrahieren eines Partikels beinhaltet, bezüglich dessen die aus dem entsprechenden Fluoreszenzbild erhaltenen Informationen und Informationen, die aus dem entsprechenden Hellfeld-Bild erhalten wurden, in dem Bereich enthalten sind, der den Maulbeerkörpern entspricht.

6. Partikeldetektionsverfahren nach Anspruch 5, wobei
die aus dem entsprechenden Hellfeld-Bild erhaltenen Informationen mindestens einen von einem Wert, der sich auf eine Größe des Partikels basierend auf dem Hellfeld-Bild bezieht, und einem Wert, der sich auf einen Fokuszustand des Partikels basierend auf dem Hellfeld-Bild bezieht, beinhalten.

7. Partikeldetektionsverfahren nach Anspruch 5 oder 6, wobei
die aus dem entsprechenden Hellfeld-Bild erhaltenen Informationen mindestens einen von einem Wert, der sich auf einen Kontrast des Partikels basierend auf dem Hellfeld-Bild bezieht, und einem Wert, der sich auf eine Variation einer Luminanz des Partikels basierend auf dem Hellfeld-Bild bezieht, beinhalten.

8. Partikeldetektionsverfahren nach Anspruch 5, wobei
das Fluoreszenzbild ein erstes Fluoreszenzbild, das durch Aufnehmen einer durch den ersten Fluoreszenzfarbstoff erzeugten Fluoreszenz erhalten wird, und ein zweites Fluoreszenzbild, das durch Aufnehmen einer durch den zweiten Fluoreszenzfarbstoff erzeugten Fluoreszenz erhalten wird, beinhaltet, und
das Extrahieren des Partikels beinhaltet
einen ersten Extraktionsschritt (S4) des Extrahierens eines Partikels, bezüglich dessen mindestens einer von einem Wert, der sich auf eine Größe des Partikels basierend auf dem Hellfeld-Bild bezieht, und einem Wert, der sich auf einen Fokuszustand des Partikels basierend auf dem Hellfeld-Bild bezieht, eine erste Bedingung erfüllt,
einen zweiten Extraktionsschritt (S5) des Erhaltens, aus dem ersten Fluoreszenzbild, eines Wertes, der sich auf eine Menge einer ersten Fluoreszenz bezieht, die durch den ersten Fluoreszenzfarbstoff von dem Partikel erzeugt wird, des Erhaltens, aus dem zweiten Fluoreszenzbild, eines Wertes, der sich auf eine Menge einer zweiten Fluoreszenz bezieht, die durch den zweiten Fluoreszenzfarbstoff von dem Partikel erzeugt wird, und des Extrahierens eines Partikels, bezüglich dessen der Wert, der sich auf die Menge der ersten Fluoreszenz bezieht, und der Wert, der sich auf die Menge der zweiten Fluoreszenz bezieht, eine zweite Bedingung erfüllen, und
einen dritten Extraktionsschritt (S6) des Extrahierens eines Partikels, bezüglich dessen mindestens einer von einem Wert, der sich auf einen Kontrast des Partikels basierend auf dem Hellfeld-Bild bezieht, und einem Wert, der sich auf eine Variation einer Luminanz des Partikels basierend auf dem Hellfeld-Bild bezieht, eine dritte Bedingung erfüllt.

9. Partikeldetektionsverfahren nach Anspruch 8, wobei
der erste Extraktionsschritt (S4), der zweite Extraktionsschritt (S5) und der dritte Extraktionsschritt (S6) derart sind, dass die Extraktion in Bezug auf eine Gruppe von Partikeln ausgeführt wird, die durch einen zuvor ausgeführten der Extraktionsschritte extrahiert wurden.

10. Partikeldetektionsverfahren nach Anspruch 9, wobei
der erste Extraktionsschritt (S4) das Extrahieren eines Partikels beinhaltet, bezüglich dessen zumindest der Wert, der sich auf die Größe des Partikels bezieht, die erste Bedingung erfüllt, und
der erste Extraktionsschritt (S4) vor dem zweiten Extraktionsschritt (S5) und dem dritten Extraktionsschritt (S6) ausgeführt wird.

11. Partikeldetektionsverfahren nach Anspruch 1, wobei
der Bereich, der den Maulbeerkörpern entspricht, ein Bereich ist, der weder mit einem Bereich, der roten Blutkörperchen entspricht, noch mit einem Bereich, der Pilzen entspricht, überlappt.

12. Partikeldetektionsverfahren nach Anspruch 1, ferner umfassend Anzeigen eines Bildes des Partikels, das extrahiert wurde und das in dem Bereich enthalten ist, der den Maulbeerkörpern entspricht (S7).

13. Partikeldetektionsverfahren nach Anspruch 12, ferner umfassend Aufnehmen eines Bildes von jedem der Vielzahl von Partikeln in der Probe, um Hellfeld-Bilder zu erhalten, wobei
das Anzeigen des Bildes des Partikels das Anzeigen des Hellfeld-Bildes des extrahierten Partikels beinhaltet.

14. Partikeldetektionsvorrichtung (1), umfassend
eine Probenvorbereitungseinheit (18), die konfiguriert ist, um eine Probe vorzubereiten, die eine von einem Subjekt gesammelte Urinprobe, einen ersten Fluoreszenzfarbstoff, der eine Nukleinsäure färbt, einen Antikörper, der spezifisch an ein rotes Blutkörperchen bindet, und einen zweiten Fluoreszenzfarbstoff, der an den Antikörper bindet, enthält;
eine Bildaufnahmeeinheit (19), die konfiguriert ist, um ein Bild von jedem einer Vielzahl von Partikeln in der Probe aufzunehmen, um Fluoreszenzbilder zu erhalten; und
eine Verarbeitungseinheit (11), die konfiguriert ist, um ein Partikel zu extrahieren, bezüglich dessen Informationen, die aus dem entsprechenden Fluoreszenzbild erhalten wurden, in einem Bereich (R2) enthalten sind, der Maulbeerkörpern entspricht.

15. Partikeldetektionsvorrichtung nach Anspruch 14, wobei
die Bildaufnahmeeinheit (19) eine Durchflusszelle (110) beinhaltet, durch die die Probe fließen gelassen wird, und
die Bildaufnahmeeinheit (19) die Vielzahl von Partikeln in der Probe abbildet, die durch die Durchflusszelle (110) fließen.

16. Programm (12a), das konfiguriert ist, um einen Computer zu veranlassen, einen Prozess zum Detektieren eines Partikels in einer von einem Subjekt gesammelten Urinprobe auszuführen, wobei der Prozess umfasst: Extrahieren eines Partikels, bezüglich dessen Informationen, die aus einem entsprechenden Fluoreszenzbild erhalten wurden, in einem Bereich (R2) enthalten sind, der Maulbeerkörpern entspricht, wobei das entsprechende Fluoreszenzbild durch Aufnehmen eines Bildes von jedem einer Vielzahl von Partikeln in einer Probe erhalten wird, die die Urinprobe, einen ersten Fluoreszenzfarbstoff, der eine Nukleinsäure färbt, einen Antikörper, der spezifisch an ein rotes Blutkörperchen bindet, und einen zweiten Fluoreszenzfarbstoff, der an den Antikörper bindet, enthält.

## Revendications

1. Procédé de détection de particules comprenant:
la préparation d'un échantillon contenant un prélèvement d'urine collecté auprès d'un sujet, un premier colorant fluorescent qui colore un acide nucléique, un anticorps qui se lie spécifiquement à un globule rouge, et un second colorant fluorescent qui se lie à l'anticorps (S1); l'imagerie de chacune d'une pluralité de particules dans l'échantillon, pour obtenir des images de fluorescence (S3); et l'extraction d'une particule concernant laquelle des informations obtenues à partir de l'image de fluorescence correspondante sont incluses dans une plage (R2) correspondant à des corps mûriformes (S5).

2. Procédé de détection de particules selon la revendication 1, dans lequel les informations obtenues à partir de l'image de fluorescence correspondante incluent une valeur relative à une quantité d'une première fluorescence générée à partir de la particule par le premier colorant fluorescent, et une valeur relative à une quantité d'une seconde fluorescence générée à partir de la particule par le second colorant fluorescent.

3. Procédé de détection de particules selon la revendication 2, dans lequel l'image de fluorescence inclut une première image de fluorescence obtenue par imagerie de la fluorescence générée par le premier colorant fluorescent, et une seconde image de fluorescence obtenue par imagerie de la fluorescence générée par le second colorant fluorescent.

4. Procédé de détection de particules selon la revendication 2, dans lequel l'extraction de la particule inclut l'extraction d'une particule concernant laquelle la valeur relative à la quantité de la première fluorescence et la valeur relative à la quantité de la seconde fluorescence satisfont une condition prédéterminée.

5. Procédé de détection de particules selon la revendication 1, comprenant en outre l'imagerie de chacune de la pluralité de particules dans l'échantillon, pour obtenir des images en champ clair, dans lequel l'extraction de la particule inclut l'extraction d'une particule concernant laquelle les informations obtenues à partir de l'image de fluorescence correspondante et des informations obtenues à partir de l'image en champ clair correspondante sont incluses dans la plage correspondant aux corps mûriformes.

6. Procédé de détection de particules selon la revendication 5, dans lequel les informations obtenues à partir de l'image en champ clair correspondante incluent au moins l'une parmi une valeur relative à une taille de la particule basée sur l'image en champ clair, et une valeur relative à un état de focalisation de la particule basée sur l'image en champ clair.

7. Procédé de détection de particules selon la revendication 5 ou 6, dans lequel les informations obtenues à partir de l'image en champ clair correspondante incluent au moins l'une parmi une valeur relative à un contraste de la particule basée sur l'image en champ clair, et une valeur relative à une variation d'une luminance de la particule basée sur l'image en champ clair.

8. Procédé de détection de particules selon la revendication 5, dans lequel l'image de fluorescence inclut une première image de fluorescence obtenue par imagerie d'une fluorescence générée par le premier colorant fluorescent, et une seconde image de fluorescence obtenue par imagerie d'une fluorescence générée par le second colorant fluorescent, et l'extraction de la particule inclut une première étape d'extraction (S4) consistant à extraire une particule concernant laquelle au moins l'une parmi une valeur relative à une taille de la particule basée sur l'image en champ clair et une valeur relative à un état de focalisation de la particule basée sur l'image en champ clair satisfait une première condition, une deuxième étape d'extraction (S5) consistant à obtenir, à partir de la première image de fluorescence, une valeur relative à une quantité d'une première fluorescence générée à partir de la particule par le premier colorant fluorescent, à obtenir, à partir de la seconde image de fluorescence, une valeur relative à une quantité d'une seconde fluorescence générée à partir de la particule par le second colorant fluorescent, et à extraire une particule concernant laquelle la valeur relative à la quantité de la première fluorescence et la valeur relative à la quantité de la seconde fluorescence satisfont une seconde condition, et une troisième étape d'extraction (S6) consistant à extraire une particule concernant laquelle au moins l'une parmi une valeur relative à un contraste de la particule basée sur l'image en champ clair et une valeur relative à une variation d'une luminance de la particule basée sur l'image en champ clair satisfait une troisième condition.

9. Procédé de détection de particules selon la revendication 8, dans lequel la première étape d'extraction (S4), la deuxième étape d'extraction (S5) et la troisième étape d'extraction (S6) sont telles que l'extraction est exécutée par rapport à un groupe de particules ayant été extraites par l'une des étapes d'extraction exécutées précédemment.

10. Procédé de détection de particules selon la revendication 9, dans lequel la première étape d'extraction (S4) inclut l'extraction d'une particule concernant laquelle au moins la valeur relative à la taille de la particule satisfait la première condition, et la première étape d'extraction (S4) est exécutée avant la deuxième étape d'extraction (S5) et la troisième étape d'extraction (S6).

11. Procédé de détection de particules selon la revendication 1, dans lequel la plage correspondant aux corps mûriformes est une plage qui ne chevauche ni une plage correspondant à des globules rouges ni une plage correspondant à des champignons.

12. Procédé de détection de particules selon la revendication 1, comprenant en outre l'affichage d'une image de la particule qui a été extraite et qui est incluse dans la plage correspondant aux corps mûriformes (S7).

13. Procédé de détection de particules selon la revendication 12, comprenant en outre l'imagerie de chacune de la pluralité de particules dans l'échantillon, pour obtenir des images en champ clair, dans lequel l'affichage de l'image de la particule inclut l'affichage de l'image en champ clair de la particule extraite.

14. Appareil de détection de particules (1) comprenant une unité de préparation d'échantillon (18) configurée pour préparer un échantillon contenant un prélèvement d'urine collecté auprès d'un sujet, un premier colorant fluorescent qui colore un acide nucléique, un anticorps qui se lie spécifiquement à un globule rouge, et un second colorant fluorescent qui se lie à l'anticorps; une unité d'imagerie (19) configurée pour imager chacune d'une pluralité de particules dans l'échantillon, pour obtenir des images de fluorescence ; et une unité de traitement (11) configurée pour extraire une particule concernant laquelle des informations obtenues à partir de l'image de fluorescence correspondante sont incluses dans une plage (R2) correspondant à des corps mûriformes.

15. Appareil de détection de particules selon la revendication 14, dans lequel l'unité d'imagerie (19) inclut une cellule à flux (110) à travers laquelle l'échantillon est amené à s'écouler, et l'unité d'imagerie (19) image la pluralité de particules dans l'échantillon s'écoulant à travers la cellule à flux (110).

16. Programme (12) configuré pour amener un ordinateur à exécuter un procédé de détection d'une particule dans un prélèvement d'urine collecté auprès d'un sujet, le procédé comprenant l'extraction d'une particule concernant laquelle des informations obtenues à partir d'une image de fluorescence correspondante sont incluses dans une plage (R2) correspondant à des corps mûriformes, l'image de fluorescence correspondante étant obtenue par imagerie de chacune d'une pluralité de particules dans un échantillon contenant le prélèvement d'urine, un premier colorant fluorescent qui colore un acide nucléique, un anticorps qui se lie spécifiquement à un globule rouge, et un second colorant fluorescent qui se lie à l'anticorps.
